Europäisches Patentamt

European Patent Office

Office européen des brevets

(11) Publication number: **0 206 733**
A1

# EUROPEAN PATENT APPLICATION

(21) Application number: 86304656.1

(22) Date of filing: **17.06.86**

(51) Int. Cl.⁴: **C 12 N 15/00**, C 07 K 13/00, C 07 H 21/04, C 12 N 1/20, C 12 P 21/02 // (C12R1/19, 1:185)

(30) Priority: **17.06.85 US 745524**

(43) Date of publication of application: **30.12.86 Bulletin 86/52**

(84) Designated Contracting States: **AT BE CH DE FR GB IT LI LU NL SE**

(71) Applicant: **GENEX CORPORATION, 16020, Industrial Drive, Gaithersburg Maryland 20877 (US)**

(72) Inventor: **Burns, Alexander Lee, 1012 Julian Place, Rockville, Maryland 20852 (US)**

(74) Representative: **Holmes, Michael John et al, Frank B. Dehn & Co. European Patent Attorneys Imperial House 15-19 Kingsway, London, WC2B 6UZ, (GB)**

(54) Cloned human serum albumin gene.

(57) Disclosed are a synthetic human serum albumin gene, plasmids containing the gene, and microorganisms transformed by those plasmids.

EP 0 206 733 A1

ACTORUM AG

1

## CLONED HUMAN SERUM ALBUMIN GENE

This invention relates to a method for synthesising a human serum albumin gene. This invention further relates to a plasmid containing a cloned human serum albumin gene and a microorganism transformed with such a plasmid.

Human serum albumin (sometimes referred to hereinafter as HSA) is the major protein component of plasma. The protein is produced in the liver and is primarily responsible for maintaining normal osmolarity in the bloodstream. It also is capable of binding and transporting various small molecules via the blood. HSA is administered in various clinical situations. Shock and burn victims, for instance, usually require doses of HSA to restore blood volume and thus ameliorate some of the symptoms associated with trauma. Persons suffering from hypoproteinemia or erythroblastosis fetalis also are likely to require treatment with serum albumin.

To date, HSA is produced primarily as a by-product from the fractionation of donated blood. A drawback to this is that the cost and supply of blood can vary widely. The blood also may contain undesirable agents such as hepatitis virus. It therefore would be advantageous to develop an alternative source of HSA.

It accordingly is an object of this invention to produce human serum albumin in microorganisms. It is a further object of this invention to so produce HSA economically. It also is an object of this invention to

2

develop a cloning procedure that can be applied to other serum proteins.

## Brief Description of the Figures

Figure 1 shows a partial restriction map of a full-length HSA cDNA clone isolated by the procedures described herein.

Figure 2 shows the DNA sequence of the 5'→3' strand of the non-coding and coding regions of the full length HSA cDNA, as well as the amino acid sequence specified by the DNA sequence.

Figure 3 shows an $A_{260}$ profile of sucrose gradient fractions of mRNA. Fraction group B was used as the template in the synthesis of HSA cDNA.

Figure 4 shows pGX401, a recombinant plasmid containing a full length HSA cDNA insert.

Figure 5 shows the DNA sequence in the region of codon 97 for HSA sequences derived from three different human livers.

According to one aspect of the present invention, we provide a synthetic human serum albumin gene. The term "synthetic" as used herein should be understood to include DNA sequences produced by use of recombriant DNA techniques and/or chemical synthesis.

In accordance with the present invention, a novel human serum albumin (HSA) gene has been cloned and bacterial expression of the gene is described. The nucleotide sequence of the full length HSA gene and the amino acid sequence of the polypeptide specified by that gene also are reported herein.

The procedure more fully described hereinafter which has been used to prepare an HSA-producing microorganism can be divided into the following stages: (1) obtaining HSA mRNA from a suitable source, e.g. by recovery and isolation of the HSA mRNA from HSA producing cells, (2) in vitro synthesis of complementary DNA (cDNA), using the mRNA as a template and conversion of the cDNA to the double-stranded form and (3) insertion of the double-stranded

- 3 -

cDNA into a suitable cloning vector and transformation of microbial cells with that cloning vector. The procedures described herein resulted in the preparation of a "full-length" cloned HSA cDNA.

Eukaryotic genes are contained in the chromosomal DNA of cell nuclei. This chromosomal DNA exists in a compact nucleoprotein complex called chromatin. Eukaryotic chromosomal DNA contains intervening sequences (introns) within the coding sequences (exons), which would not permit correct expression in bacteria. For this reason a preferred method for producing contiguous coding blocks of a particular protein involves the use of messenger RNA (mRNA). Messenger RNA has a ribonucleotide sequence corresponding to the gene of interest without the introns and conveniently can be recovered from eukaryotic cells that produce the protein specified by the gene.

Human serum albumin mRNA can be recovered in useful quantities from human liver cells. The HSA mRNA produced by the liver cells is complementary to one of the two strands of the HSA gene and may be employed as a template for the synthesis of complementary DNA (cDNA) as hereinafter described. To effectively utilize the mRNA for the synthesis of cDNA, it advantageously is recovered from the cells in relatively pure form. The guanidine thiocyanate/guanidine hydrochloride extraction procedure described by McCandliss et al., Methods in Enzymology 79:51 (1981), advantageously may be used to recover and purify the HSA mRNA. RNA is inherently less stable than DNA, and is particularly subject to degradation by ribonucleases that are present in the cells. Therefore, mRNA recovery procedures generally employ means for rapidly inactivating any ribonucleases which are present.

In general, recovery of total RNA is initiated by disrupting the cells in the presence of a ribonucleaseinactivating substance. Disruption of the cells may be

4

accomplished by subjecting the cells to a lysing reagent, freezing/thawing, or mechanical disruption; preferably a combination thereof. A mixture of guanidine thiocyanate and a reducing agent, such as mercaptoethanol, has been found to function effectively as a ribonuclease inactivator (McCandliss, et al., supra).

After disruption of the cells, the solid cell debris is removed, e.g. by centrifugation, and the RNA is precipitated from the resulting clarified solution. Precipitation is effected by known techniques, such as adding a water-miscible alcohol, e.g. ethanol, to the solution in a precipitating amount. The RNA then is resuspended in a guanidine hydrochloride solution and precipitated with ethanol for two successive cycles. At this point the RNA is undegraded and free of proteins and DNA.

The next step is the separation of mRNA from the total precipitated RNA. Human serum albumin mRNA is polyadenylated, therefore, it readily can be separated from non-adenylated RNA by affinity chromatography with oligodeoxythymidylate (oligo dT) cellulose (Aviv, H., et al., Proc. Natl. Acad. Sci. USA 69: 1408 (1972); McCandliss, et al., supra). Total RNA can be applied to a column in an approximately 0.5 M NaCl containing solution. Under these conditions only poly A+ RNA binds to the oligo dT cellulose and can be removed specifically by washing the column in a salt free solution.

To enrich the preparation for HSA mRNA, the poly $A^+$RNA can be fractionated according to size by sucrose gradient centrifugation. Activity of the RNA in the various gradient fractions can be verified by in vitro translation in a reticulocyte lysate (Pelham, H., et al. Eur. J. Biochem. 67:247 (1976)) and by electrophoretic analysis of the protein products (Laemmli, U., Nature 227:680 (1970)).

5

Once a poly A⁺RNA fraction able to synthesize proteins the size of HSA has been isolated, it can be used to provide a template for cDNA synthesis. This procedure involves enzymatically constructing double-stranded DNA, which has a nucleotide base pair sequence identical to the coding sequence of the original chromosomal gene. The cDNA does not contain any noninformational segments (introns) within the coding region which might be present in the eukaryotic gene, and thus can ultimately be transcribed and translated in prokaryotic systems.

Synthesis of HSA cDNA employs the enzymes reverse transcriptase, Klenow fragment of DNA polymerase I and S1 nuclease (Kacian, D., et al., Proc. Nat. Acad. Sci. USA 73:2191 (1976); McCandliss, R., et al., Methods in Enzymology 79, p. 601 (1981)). Reverse transcriptase catalyzes the synthesis of a single strand of DNA from deoxynucleoside triphosphates on the mRNA template. The poly r(A) tail of the mRNA permits oligo (dT) (of about 12 to 18 nucleotides) to be used as a primer for cDNA synthesis. The use of a radioactively-labelled deoxynucleoside triphosphate facilitates monitoring of the synthesis reaction. Generally, a $^{\alpha 32}$P-containing deoxynucleoside triphosphate advantageously may be used for this purpose. The cDNA synthesis generally is conducted by combining the mRNA, the deoxynucleoside triphosphates, the oligo (dT) and the reverse transcriptase in a buffered solution. This solution is incubated at an elevated temperature, e.g., about 40-50°C, for a time sufficient to allow formation of the cDNA copy, e.g. about 5-20 minutes. The conditions of the reaction are essentially as described by Kacian, D.L., et al., supra. After incubation, disodium ethylenediaminetetraacetic acid (hereinafter EDTA) is added to the solution, and the solution is extracted with phenol:chloroform (1:1 by

6

vol.). The aqueous phase is advantageously purifed by gel filtration chromatography, and the cDNA-mRNA complex in the eluate is precipitated with alcohol.

The mRNA can be selectively hydrolyzed in the presence of the cDNA with dilute sodium hydroxide (about 0.1 M) at an elevated temperature, e.g., about 60-80°C for about 15-30 minutes. Neutralization of the alkaline solution and alcohol precipitation yields a single-stranded cDNA copy.

The single-stranded cDNA copy has been shown to have a 5'-poly (dT) tail, and to have a 3' terminal hairpin structure, which provides a short segment of duplex DNA (Efstratiadis, A., et al., Cell, 7, 279 (1976)). This 3' hairpin structure can act as a primer for the synthesis of a complementary DNA strand. Synthesis of this complementary strand is conducted using the Klenow fragment of DNA polymerase I (Klenow, H., et al., Eur. J. Biochem., 22, 371 (1971)) in a reaction mixture containing the deoxynucleoside triphosphates. The duplex cDNA recovered by this procedure has a 3' loop, resulting from the 3' hairpin structure of the single-stranded cDNA copy. This 3' loop can be cleaved by digestion with the enzyme, S1 nuclease, using essentially the procedure of McCandliss et al., Methods in Enzymology 79:601 (1981). The S1 nuclease digest may be extracted with phenolchloroform, and the resulting cDNA precipitated from the aqueous phase with alcohol.

The intact double-stranded DNA (about 2000 base pairs) corresponding to a human serum albumin gene can be isolated by, for example, sucrose gradient centrifugation, using the procedure of McCandliss supra p. 51. In order to determine the sizes of the DNA in the sucrose gradient, aliquots of the gradient fractions are electrophoresed in a polyacrylamide gel with molecular weight markers. The resulting gel is first stained with ethidium bromide to visualize the markers and then autoradiographed to detect the radioactive cDNA. The fractions of the gradient containing DNA molecules larger than 1000 base pairs are pooled and the DNA is precipitated with ethanol.

For purposes of amplification and selection, the double-stranded cDNA gene prepared as described above is generally inserted into a suitable cloning vector, which is used for transforming appropriate host cells. Suitable cloning vectors include various plasmids and phages, with plasmids being preferred in this case. The criteria for selecting a cloning vector include its size, its capability for replicating in the host cells, the presence of selectable genes, and the presence of a site for insertion of the gene. With respect to its size, the vector is advantageously relatively small, to permit large gene insertions, and so as not to divert large amounts of cellular nutrients and energy to the production of unwanted macromolecules. The vector also includes an intact replicon which remains functional after insertion of the gene. This replicon preferably directs the desired mode of replication of the plasmid, i.e., multiple copies or a single copy per cell, or a controllable number of copies per cell. Genes specifying one or more phenotypic properties, preferably antibiotic resistance, facilitate selection of transformants. The insertion site is advantageously a unique restriction site for a restriction endonuclease. A cloning vector meeting all of these criteria is the plasmid pBR322. The cDNA can be conveniently inserted into this plasmid by a homopolymeric tailing technique. Homopolymer tails are added to the 3'-hydroxyl groups of the human serum albumin double-stranded cDNA gene, by reaction with an appropriate deoxynucleoside triphosphate, in the presence of terminal deoxynucleotidyl transferase. The plasmid is

8

opened by digestion with the appropriate endonuclease,and complementary homopolymer tails are added to the 3'-hydroxyl groups of the opened plasmid, using the homopolymeric tailing technique. Appropriate reaction conditions have been described for the addition of dC residues to ds cDNA (McCandliss, R., et al., page 601 supra; Roychoudhury, R., et al., Nucleic Acids Research 3:101 (1976)) and of dG residues to PstI treated pBR322 (Maeda, S., Methods in Enzymology 79:607 (1981)). In a preferred embodiment, however, the molar excess of dXTPs to 3' ends is in the range of 3000 to 5000. Progress of the reactions is monitored until the chain length is approximately 15. The tailed cDNA and plasmids are recovered, e.g., by phenol extraction followed by alcohol precipitation. The homopolymeric ends of the two DNAs are complementary and will anneal together under appropriate conditions to yield a recombinant plasmid containing the HSA gene (Maeda, S., Methods in Enzymology 79:611 (1981)).

A suitable strain of E.coli may be transformed with this recombinant plasmid, using essentially the method of Lederberg, J. Bacteriology 119:1072 (1974) and be maintained indefinitely.

Generally, several hundred to several thousand clones are produced by these procedures and can be screened for the presence of the HSA gene with, for example, rat serum albumin cDNA. A nick translated (Maniatis, T., et al., Proc. Natl. Acad. Sci. USA 72:3961 (1975)) rat cDNA having 85% homology with human cDNA can be used to hybridize to plasmid cDNA attached to nitrocellulose filters (Grunstein, M., et al., Proc. Natl. Acad. Sci. USA 72:396 (1975), Southern, E.M. J. Mol. Biol., 98:503 (1975)). In this procedure, DNA from each colony (or from groups of colonies) is fixed to discrete zones of a nitrocellulose filter and denatured.

Alternatively, the DNA can be electrophoresed in a gel prior to fixing on a filter. A solution of the radioactively labeled rat cDNA is applied thereto under hybridizing conditions. Unhybridized rat cDNA is washed from the filter, and colonies containing DNA to which the rat cDNA hybridized are identified by autoradiography. One positive clone was identified but found to be an incomplete HSA cDNA by DNA sequencing. A portion of this HSA cDNA was then nick translated in order to rescreen the entire bank of clones. Ninety positive hybridization signals were thus obtained.

Positive clones may be cultivated on suitable growth media to obtain ample quantities of cells from which to extract the plasmid DNA. The plasmid DNA is extracted, using conventional techniques, such as disruption of the cells, followed by phenol extraction, and alcohol precipitation. The plasmid and chromosomal DNAs may be separated, e.g. by electrophoresis or cesium chloride equilibrium centrifugation. Plasmid DNA containing inserts of about 1500 to 2000 base pairs are selected for further characterization.

The cloned gene can be excised from the plasmid DNA and then characterized by sequencing analysis (Sanger, F., et al., Proc. Natl. Acad. Sci USA 74:5463 (1977); Maxam, A., et al., Proc. Natl. Acad. Sci. USA 74:560 (1977)).

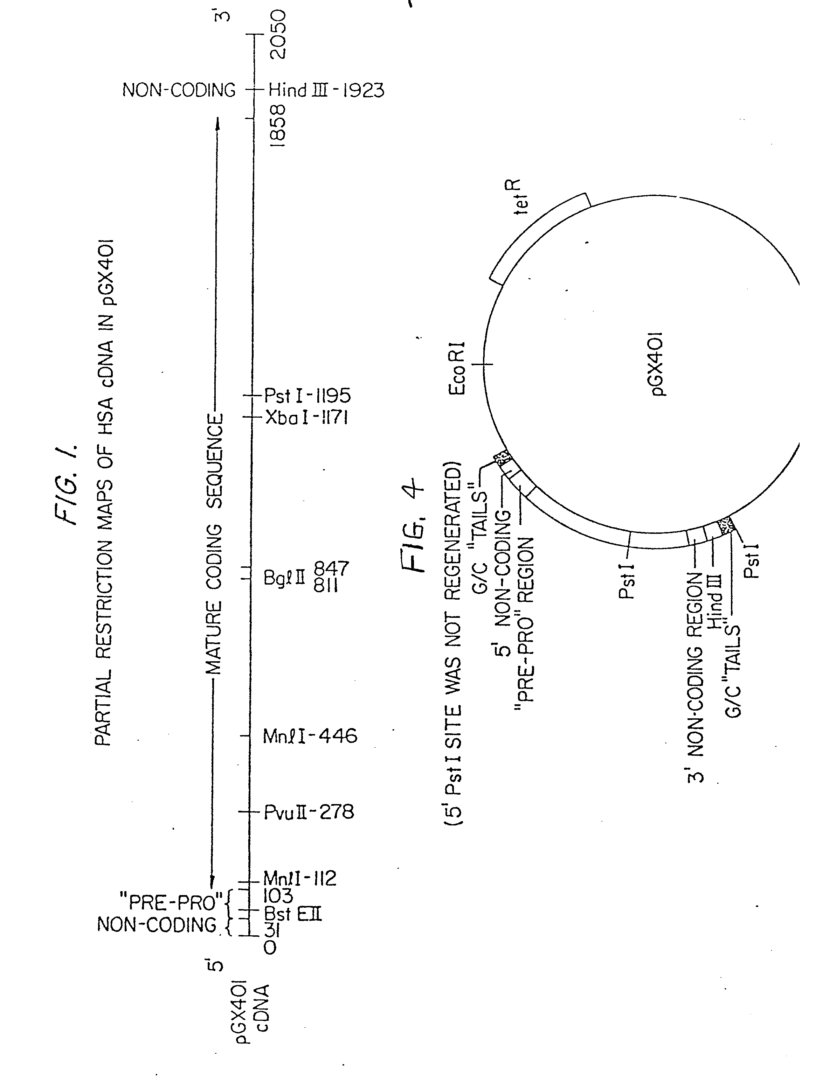

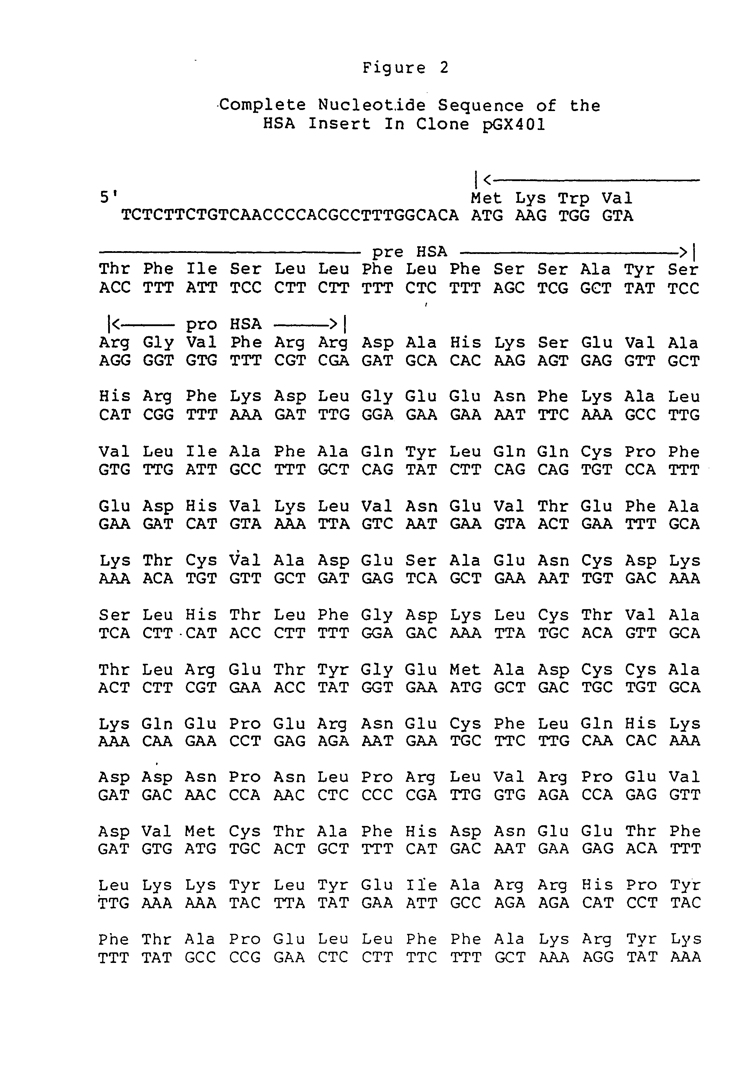

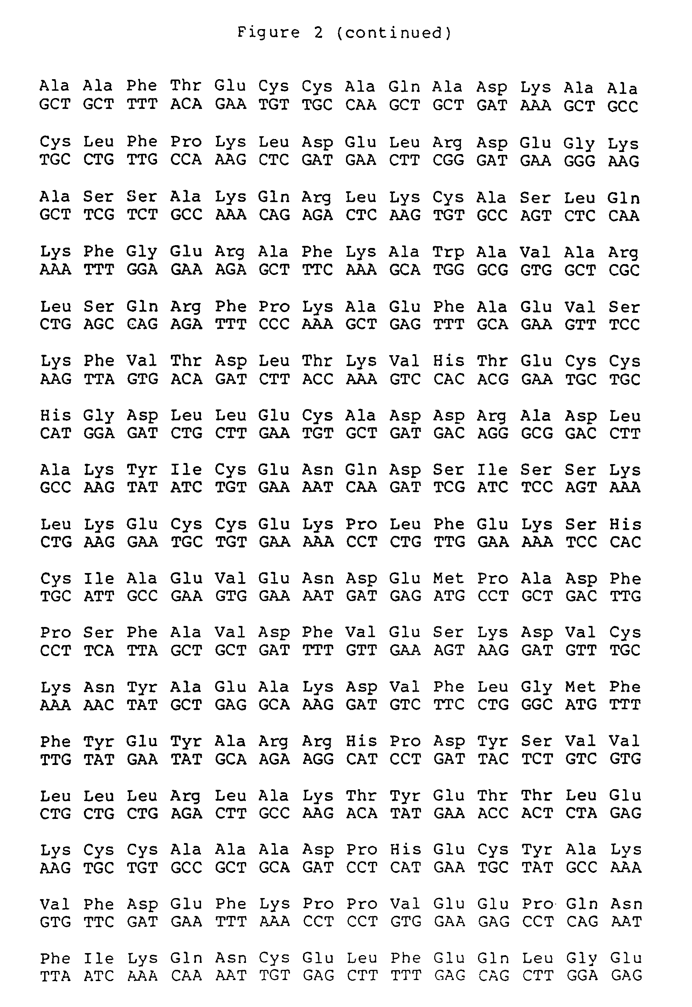

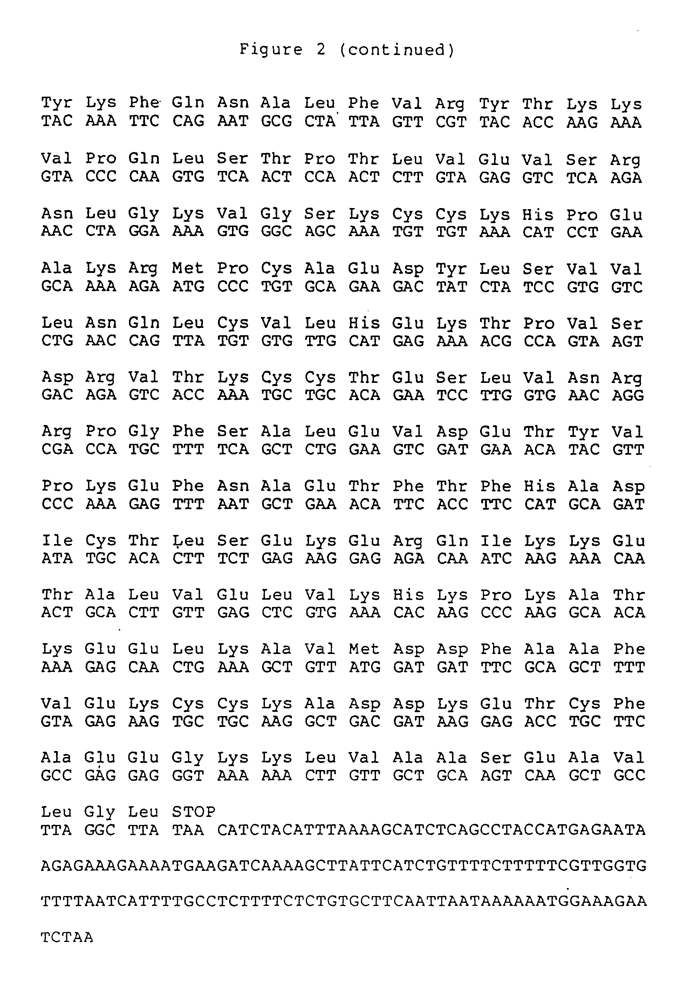

By these procedures a prepro-HSA clone has been isolated. An E. coli HB101 culture transformed with the plasmid containing this prepro-HSA gene has been deposited with the U.S. Department of Agriculture Northern Regional Research Laboratory in Peoria, Illinois, as NRRL No. B-15784. A diagnostic partial restriction map of this HSA gene insert is shown in Figure 1 of the drawings and Figure 2 shows the 5'-->3'

10

strand of the non-coding and coding regions, along with the amino acid sequence specified by the gene.

The cloned prepro-HSA coding sequence consists of 2050 base pairs excluding the oligo dC tails added to the cDNA. The gene has noncoding regions at the 5' end (base pairs 1-31) and at the 3' end (base pairs 1858-2050). The 5' end of the coding region (32- 103 base pairs) includes a 24 amino-acid leader (an 18- amino-acid "pre" sequence followed by a 6-amino-acid "pro" sequence) and the mature human serum albumin protein is specified by the region from base pair number 104 to base pair number 1858.

As used in Figure 2 and elsewhere herein, the abbreviations have the following standard meaning:

| | | |
|-----|---|----------------|
| A | = | deoxyadenyl |
| T | = | thymidyl |
| G | = | deoxyguanyl |
| C | = | deoxycytosyl |
| GLY | = | glycine |
| ALA | = | alanine |
| VAL | = | valine |
| LEU | = | leucine |
| ILE | = | isoleucine |
| SER | = | serine |
| THR | = | threonine |
| PHE | = | phenylalanine |
| TYR | = | tyrosine |
| TRP | = | tryptophan |
| CYS | = | cysteine |
| MET | = | methionine |
| ASP | = | aspartic acid |
| GLU | = | glutamic acid |
| LYS | = | lysine |
| ARG | = | arginine |
| HIS | = | histidine |
| PRO | = | proline |

11

        GLN   =   glutamine

        ASN   =   asparagine

It will be appreciated that because of the degeneracy of the genetic code, the nucleotide sequence of the gene can vary substantially.  For example, portions or all of the gene could be chemically synthesized to yield DNA having a different nucleotide sequence than that shown in Figure 2, yet the amino acid sequence would be preserved, provided that the proper codon-amino acid assignments were observed.  Having established the nucleotide sequence of the human serum albumin gene and the amino acid sequence of the protein, the gene of the present invention is not limited to a particular nucleotide sequence, but includes all variations thereof as permitted by the genetic code.

It is believed that the amino acid sequence set forth in Figure 2 and claimed herein represents a genomic HSA allele that is widespread in the human population, in contrast to the sequences previously published in the scientific literature.  Polymorphism is known for HSA. Protein electrophoresis has revealed over twenty genetic variants of HSA (Weitkamp et al., _Ann. Hum. Genet. London_ 36:381 (1973)).  Two differing amino acid sequences have been reported previously.  See Lawn, R.M., et al., _Nucl. Acids Res._ 9:6103 (1981) and Dugiaczyk, A., et al., _PNAS_ 79:71 (1982).  The DNA sequence of Figure 2 differs from each of these published sequences. Although some of the differences occur in third base position of codons or in the noncoding regions, and as such do not cause amino acid changes, conflicting nucleotide sequence data suggest different amino acids at positions 97 and 396.  In Figure 2, the amino acid represented by codon 97 (GAG) is glutamic acid.  The same was reported by Lawn, et al., _supra_. Dugiaczyk, however, reported that codon to be GGG (glycine).  Codon 396 in

12

Figure 2, also is designated GAG (gluatmic acid). Dugiaczyk reported the same; however, Lawn reported codon 396 to be AAG (lysine). Thus, each of the three DNA sequences would encode a different polypeptide. Example IV below sets forth the procedures followed to determine that these differences represented true protein polymorphism and not merely experimental artificats.

The present invention has been described in connection with the use of E. coli as the bacterial host for recombinant DNA containing the HSA gene, but skilled molecular biologists will appreciate that other gram-negative bacteria, such as Pseudomonas; gram-positive bacteria, such as Bacillus; higher unicellular organisms, such as yeasts and fungi, and mammalian cells can be employed for cloning and/or expression of the HSA gene.

The invention is further illustrated by reference to the following examples, which are not intended to be limiting.

## EXAMPLE I
### Isolation of HSA mRNA from Human Liver Tissue

Messenger RNA (mRNA) was isolated from human liver tissue taken from a 10-year-old accident victim. Extreme care was taken throughout the procedures to avoid ribonuclease contamination of the mRNA preparation. These measures included the use of new, sterile laboratory glassware, treatment of solutions with diethylpyrocarbonate when appropriate, followed by autoclaving, keeping the preparation cold when possible and using gloves to avoid contact of the preparation with skin.

Frozen human liver tissue (10.5 grams) was homogenized in 210 mls lysis solution (4M guanidine thiocyanate/0.1M Tris-HCl, pH 7.5/0.1M 2-mercaptoethanol) using a Virtis homogenizer. Cellular debris was pelleted by centrifugation at 8750 rpm, 4°C, for 10 minutes in a Sorvall GSA rotor, and the supernatant was transferred to a new centrifuge bottle. To the supernatant were added 0.04 volume 1M acetic acid and 0.5 volume 95% ethanol. After 2 hours at -20°C, the mixture was centrifuged at 7500 rpm, 10 minutes, 4°C and the pellet resuspended in 50 mls wash solution (6M guanidine hydrochloride/10mM Na$_2$·EDTA, pH 7.0/10mM dithiothreitol.) Centrifugation at 5500 rpm, 10 minutes, pelleted particulate debris, and the supernatant was transferred to a new centrifuge bottle. To the supernatant were added 0.04 volume 1M acetic acid and 0.5 volume 95% ethanol. After 2 hours at -20°C, the mixture was centrifuged at 7200 rpm 20 minutes. The pellet was resuspended in 20 mls wash solution, and 0.04 volume 1M acetic acid and 0.5 volume 95% ethanol were added. The mixture was kept at -20°C for 12 hours, then centrifuged at 8,000 rpm for 10 minutes at 4°C in a Sorvall SS-34 rotor. The pellet was resuspended in 15 mls sterile distilled H$_2$O (dH$_2$O) and extracted with an equal volume of (4:1) chloroform: butanol. The aqueous phase was transferred to a fresh tube and 0.1 volume 2.4 M sodium acetate and 2.5 volumes 95% ethanol were added. After 2.5 hours at -20°C, the RNA was pelleted by centrifugation and the pellet was resuspended in 2 mls sterile d H$_2$0). A total of 19.2 mg RNA was recovered.

mRNA was then separated from the total RNA using generally, the oligo(dT)-cellulose affinity chromatography procedure described in Aviv et al.. supra and McCandliss, et al., supra. A column of 5 grams oligo(dT)-cellulose was washed with one column volume 0.1M NaOH to denature any ribonuclease present, then equilibrated with high salt buffer (10mM Tris-HCl, pH 7.4/0.5M NaCl/0.5% sodium dodecyl sulfate). The total RNA preparation, dissolved in two mls dH$_2$O above, was

heated at 70°C for 1 minute, then cooled on ice to room temperature. Next, 0.1 volume 5M NaCl, 0.04 ml 0.5M Tris-HCl, pH 7.5, and 0.1 ml 10% sodium dodecyl sulfate (SDS) were added to the RNA. 8 mls high salt buffer were then added to the RNA and the solution was applied to the column with a flow rate of about 10 drops/minute. After the sample had passed through, unbound RNA was washed from the column with high salt buffer. Fractions (1/2 ml each) were collected and the optical density at 260 nm ($A_{260}$) of each fraction was measured in a spectrophotometer. The column was washed until the $A_{260}$ readings of fractions dropped below 0.05. Undesired RNA was further washed from the column with low salt buffer (10mM Tris-HCl, pH 7.4/0.2M NaCl/0.1% SDS) and fractions were collected as above until the $A_{260}$ had dropped to 0.05.

Next, the mRNA was eluted from the column with elution buffer (10mM Tris-HCl, pH 7.4/1mM EDTA/0.1% SDS) and 1 ml fractions were collected until the $A_{260}$ was less than 0.05. The first 15 fractions (those having the highest $OD_{260}$ readings) were pooled and the mRNA was precipitated by adding 0.1 volume 2.4M sodium acetate and 2.5 volumes 95% ethanol, and placing at -20°C for 12 hours. The eluted mRNA was then pelleted by centrifugation and resuspended in 800 µl elution buffer. After heating the resuspended pellet at 70°C for 90 seconds then cooling on ice; 0.1 volume 5M NaCl and 0.05 volume 10% SDS were added.

The eluted mRNA prepared above was then further purified by passage over a second oligo(dT)-celluose column. A column containing 0.1 gram oligo(dT) cellulose was washed with NaOH, then with high salt buffer as previously described. The RNA was applied to the column and fractions were collected with high salt, low salt, and elution buffers as with the first column. The peak fractions from the elution buffer step were pooled and the twice-purified mRNA was precipitated and pelleted as before.

The mRNA was then size-fractionated on a 12-ml sucrose gradient as described in McCandliss et al., Methods in Enzymology, 79, pp. 56-58. A 5-20% sucrose gradient was prepared in gradient buffer (0.02M sodium acetate, pH 5.6) and chilled at 4°C for 3 hours. 100µg of the mRNA was resuspended in 100µl gradient buffer, heated at 80°C for 2 minutes, quick-cooled in an ice bath, then layered on top of the gradient. A second 5-20% gradient had E. coli 16 and 23S rRNA (100µg total) loaded on it to serve as molecular weight markers.

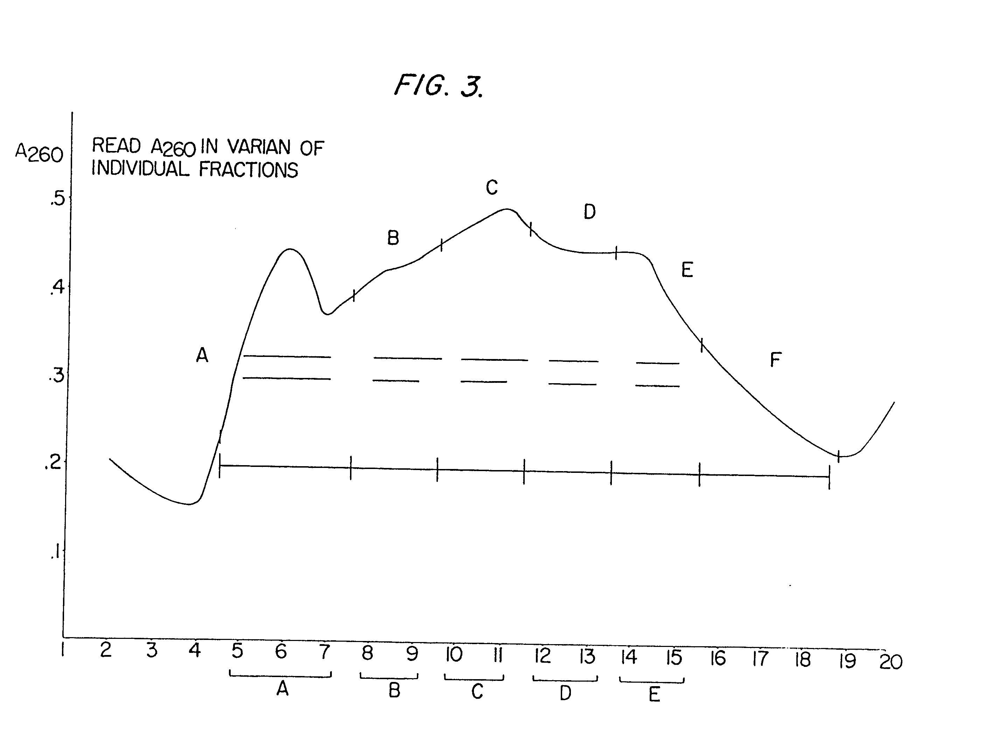

The two gradients were centrifuged in a Beckman SW40 rotor at 38,000 rpm for 12.5 hr at 4°C. Fractions of about 0.5 ml were then collected and the $A_{260}$ measured (fraction #1 is that collected from the bottom of the gradient tube.) The $A_{260}$ peak was divided into 6 groups of fractions, groups A through F as shown in Figure 3. The fractions in each group were pooled and the mRNA precipitated with 0.1 volume 2.4 M sodium acetate and 2.5 volumes 95% ethanol.

Fraction groups containing mRNA which encodes protein of the size expected for HSA were identified by in vitro translation using a rabbit reticulocyte lysate kit (available from Bethesda Research Laboratories and used according to manufacturer's instructions) supplemented with $^{35}S$ methionine. A reaction mixture for each fraction group contained the components necessary for translation of the mRNA into radioactively-labeled proteins which were visualized by electrophoresis on a 12.5% polyacrylamide/SDS gel, followed by fluorography.

The fluorogram showed a prominent protein band of the size expected for HSA (68,000 daltons) among the translation products of fraction groups B and C. Group B

16

had a much lower percentage of protein products in undesirable low molecular weight range so the mRNA in group B was chosen for use as a template in the synthesis of cDNA.

## EXAMPLE II
### Synthesis of HSA cDNA

Generally, the cDNA synthesis procedure of McCandliss et al., Methods in Enzymology, 79, pp. 601-607 (1981) was used. Incorporation of a radioactively labeled deoxynucleotide allowed monitoring of the synthesis and calculation of yields at each step.

The first strand of cDNA was synthesized on the mRNA template, using oligo-dT as a primer, as follows.

Prepared mix and kept on ice:

| | |
|---|---|
| 0.5 M Tris-HCl, pH 8.3 | 20μl |
| 1.4 M KCl | 10μl |
| 0.25M MgCl$_2$ | 8μl |
| 0.05M dATP, pH 7.0 | 2μl |
| 0.05M TTP, pH 7.0 | 2μl |
| 0.05M dCTP, pH 7.0 | 2μl |
| 0.05M dGTP, pH 7.0 | 2μl |
| 0.01M dithiothreitol | 4μl |
| sterile distilled H$_2$O | 45μl |
| aqueous label, $\alpha^{32}$P-dCTP (10μCi/μl) | 5μl |
| | 100μl |

17

Added remaining components:

| | |
|---|---|
| oligo(dT)$_{12-18}$(250 μg/ml) | 20 μl |
| actinomycin D (500 μg/ml, aqueous) | 16 μl |
| 10μg mRNA, "B" fraction | 20 μl |
| sterile dH$_2$O | 37 μl |
| *AMV reverse transcriptase (16u/μl) | 7 μl |
| Total volume: | 200 μl |

*Avian myeloblastosis virus (AMV) reverse transcriptase is kept at -80°C and thawed briefly to add as last component

The reaction mixture was kept on ice 5 minutes and 2μl were removed and counted in ASC scintillation fluid in order to determine the specific activity of the dCTP. The reaction mixture was then incubated 10 minutes at 46°C. 20μl 0.2M EDTA pH 8.0 was added to stop the reaction, and the mixture was then extracted with an equal volume (1:1) phenol:chloroform.

0.14 volume 80% glycerol was added and sample was chromatographed on a 0.7 x 17 cm. Sephadex G-100 column. Once the sample had entered the column, G100 buffer (10mM Tris-HCl, pH 8.0/1mM EDTA/100mM NaCl) was added to the column and 5-drop (about 275μl) fractions were collected. The radioactive fractions were "Cerenkov counted" and the cDNA fractions comprising the peak counts per minute were pooled. The mRNA/cDNA hybrids were precipitated by adding 0.1 volume 2.4M sodium acetate and 2.5 volumes 95% ethanol, placing in a dry ice/ethanol bath for 30 minutes, then pelleting by centrifugation at 10,000 rpm, 4°C, for 20 minutes. The pellet was resuspended in 300μl 0.1M NaOH and heated at 70°C for 20 minutes to hydrolyze the RNA, leaving single-stranded cDNA. 30μl 1M HCl were added to neutralize the solution. The DNA was precipitated by adding 5μg tRNA, 1/10 volume 2.4M sodium acetate, and 2.5 volumes 95% ethanol, placing in a dry ice-ethanol bath 10 minutes, and centrifuging in a microfuge 10 minutes at 4°C.

The pellet was resuspended in the following mix:

    40µl  0.5M potassium phosphate, pH 7.4
     8µl  0.25M MgCl$_2$
     2µl  0.1M dithiothreitol
     1µl  0.05M dATP, pH 7.0
     1µl  0.05M dCTP, pH 7.0
     1µl  0.05M dGTP, pH 7.0
     1µl  0.05M TTP,  pH 7.0
    124µl  sterile dH$_2$O
    178µl

Next, added 22µl DNA polymerase I Klenow fragment (5µ/µl, available from Boehringer-Mannheim.)

The reaction mixture was then incubated in a 15°C water bath for 12 hours. 20µl 0.2M EDTA pH 8.0 was added to stop the reaction and the mixture was extracted with an equal volume (1:1) phenol:chloroform. 0.14 volume glycerol was added to the aqueous phase.

The sample, which now contains double-stranded cDNA, was run over a Sephadex G100 column and the peak cDNA fractions were pooled and precipitated as before. The double-stranded DNA has a 3' "hairpin loop" as previously described, which was removed with S1 nuclease as follows. The pellet was resuspended in 72 µl sterile distilled water and then 18 µl 5X S1 buffer (1M NaCl/0.25M sodium acetate, pH 4.5/5mM ZnSO$_4$/2.5% glycerol) were added. An enzyme mix was prepared by adding 2.5 µl (50 units) of S1 nuclease (20µg/µl) to 47.5 µl 1X S1 buffer. 10µl of enzyme mix was added to the 90µl DNA solution then incubated at 37°C 20 minutes. Addition of 20 µl 0.2M sodium EDTA stopped the reaction, and the reaction mixture was extracted with an equal volume (1:1) phenol:chloroform. The aqueous phase was loaded onto a 5-25% sucrose gradient and spun at 38,000 rpm 17.5 hours 5°C in an ultracentrifuge.

One-ml fractions were collected and "Cerenkov counted." Fractions were pooled with fractions 1-6, 7-9, and 10-12 comprising the 3 pools. Fraction #1 was the fraction taken from the bottom of the gradient. DNA was precipitated by adding 0.1 volume 2.4M sodium acetate, 1-2 µg tRNA, and 2.5 volumes 95% ethanol to each pool, then placing them at -20°C overnight. The DNA was pelleted by centrifugation at 25K for 30 minutes at 4°C. After slightly dessicating pellets, the DNA from each pool was resuspended in 200µl $dH_2O$ and precipitated again with ethanol and sodium acetate. Pellets were resuspended in 22µl $dH_2O$ and spun in a microfuge 5 minutes to pellet insoluble matter. 2µl of each cDNA-containing supernatant were analyzed by electrophoresis on a 6% polyacrylamide gel. Autoradiography of the gel showed that the DNA in the pool of fractions 1-6 had an average size of 1100 base-pairs (bp) and included DNA in the 200 bp range and this pool was chosen for addition of "polyC tails" to the 3' ends of the cDNA, using, generally, the homopolymeric tailing procedure described in McCandliss et al., page 601 et seq., supra. A 5000 molar excess of dCTP over 3' cDNA ends was found to give good results.

The reaction mixture was as follows:

    20µl cDNA (about 43 ng)

    -    $^3$H dCTP (645 pmol, lyophilized)

    2.4µl 10X TdT buffer*

    <u>1.6µl dH$_2$O</u>

    24.0µl

*10X TdT buffer = 1.4M potassium cacodylate/0.3M Tris-HCl, pH 7.0/10mM $CoCl_2$/1mM DTT)

The reaction mixture was preincubated to 37°C for 2 minutes, 2µl were removed for use in calculations, then 2µl (6.66 units) P-L Biochemicals terminal deoxynucleotidyl transferase were added and incubation at 37°C was continued for 5 minutes. Calculations based on incorporation of $^3H$ dCTP indicated that the 3'ends of the cDNA now carried "polyC tails" an average of 14 nucleotides in length. 80μl T.E. buffer (10mM Tris-HCl, pH 7.6/1mM EDTA) were added to the DNA and the solution was extracted with an equal volume of (1:1) phenol:chloroform. The organic phase was then retracted with 100μl $dH_2O$ and the two aqueous phases were combined.

The C-tailed double-stranded cDNA was then annealed to plasmid pBR322 DNA which had been linearized with the restriction endonuclease PstI, then "G-tailed" by the homopolymeric tailing method. The complementary single-stranded C and G "tails" will anneal, producing recombinant plasmids with cDNA inserts at the PstI site.

> 200μl cDNA, C-tailed (39.2 ng)
> 10.5μl pBR322-PstI, G-tailed (302 ng)
> 93μl 10X buffer*
> 626.5 μl $dH_2O$
> 930μl

The reaction mix was placed in an insulated water bath at 70°C. The bath was then transferred to a 37°C room and allowed to cool slowly to 37°C overnight, then transferred to room temperature, where the bath cooled to 30°C over several hours. The reaction mixture was then stored at 4°C.

*(10X annealing buffer = 1.5M NaCl/100mM Tris-HCl, pH7.5/10mM EDTA)

E. coli HB101 cells were made competent for transformation by known calcium chloride treatment procedures. 200μl aliquots of competent HB101 cells were each combined with 40μl of the annealing reaction mixture and kept on ice 20 minutes, then heat-shocked at 42°C for 2 minutes. 2.8 mls Luria broth were added to

21

each tube and incubated at 37°C for 1 hour. The tubes' contents were aliquoted (1/2 ml aliquots) into tubes containing Luria broth plus 0.7% agar, and then were poured onto Luria broth-agar plates containing 25 µg/ml tetracycline and incubated at 37°C until colonies appeared.

Only those cells transformed by pBR322 (with or without a cDNA insert) can grow on tetracycline plates. Approximately 2500 transformant colonies grew on the plates.

## EXAMPLE III
### Isolation of a Full-Length HSA cDNA

The transformants were initially screened with a rat serum albumin (RSA) cDNA fragment. The RSA cDNA fragment was obtained from a pBR322 plasmid containing a 2000 bp RSA cDNA insert. This recombinant plasmid is similar to, but contains a longer cDNA insert than, the plasmid prAlbI described in Proc. Nat'l. Acad. Sci. USA, 76, 4370 (1979). A 1480 bp rat serum albumin (RSA) fragment was isolated by digesting the plasmid carrying the RSA cDNA with the restriction endonuclease BstEII (all restriction endonucleases used in these examples were used according to manufacturer's specifications.) The fragment was then radioactively labeled with $\alpha^{32}P$ by the "nick translation" procedure (Maniatis et al. PNAS USA, 72:3961 (1975)).

About 80 10-ml cultures of individual transformants were grown and plasmid DNA was isolated by known plasmid "mini-prep" procedures. The partially purified plasmid DNAs were subjected to electrophoresis on 0.8% agarose gels. The DNA was transferred from the gels to nitrocellulose filters using the "Southern blotting"

22

technique (Southern, E.M. J. Molec. Biology 98, 503 (1975)).

The nitrocellulose filters were immersed for 2 hours at 42°C in prehybridization solution (50% formamide/5X SSC*/0.05M NaPO$_4$, pH 6.5/5X Denhardt's*/100µg/ml salmon sperm DNA). The filters were then transferred into hybridization solution (50% formamide/10% dextran sulfate/5X SSC/20mM NaPO$_4$, pH 6.5/1X Denhardt's/50µg/ml salmon sperm DNA.) The nick-translated 1480bp RSA fragment prepared above was heated at 100°C for 5 minutes, then quick cooled on ice, and this probe was added to the hybridization solution at 2 X 10$^5$ cpm probe per ml of solution. The filters were incubated in the hybridization solution at 42°C for 18 hours, then washed twice in 2XSSC and once in 0.1X SSC at room temperature.

Autoradiography of the filters revealed nonspecific hybridization of the probe to all plasmid DNAs. Therefore, several Southern blot filters were washed in 2XSSC at various temperatures from 65°C to 80°C. DNA from one plasmid on a filter washed at 65°C hybridized strongly with the probe.

DNA sequencing revealed that the "positive" clone, called 6C3, was a partial-length human serum albumin clone. Plasmid DNA was isolated from a culture of 6C3 and digested with the restriction endonuclease PstI. One of the resulting HSA cDNA fragments, about 475bp in length, was isolated and "nick translated" for use as a

*50X Denhardt's stock = 1% polyvinylpyrrolidone/1% ficoll/1% bovine serum albumin.

1XSSC = 150mM NaCl/15mM sodium citrate, pH 6.8 with citric acid

0206733

23

probe. The entire bank of approximately 2500 clones was screened with this probe using a modification of the hybridization procedure of Grunstein et al., _supra_.

The transformant colonies were individually picked from the plates into separate wells in 96-well microtiter plates containing Luria broth plus 0.2% glucose plus 25µg/ml tetracycline and incubated at 37°C ovenight. Using a transfer device with 48 metal prongs, samples of each culture were transferred to two Luria broth/agar/tetracycline plates, one plate previously overlaid with a nitrocellulose filter, and incubated at 37°C 2 days. The filters were then placed successively on Whatman filter paper soaked in one of the following solutions: 0.5M NaOH; 1MTris, pH7.4; 1M Tris, pH7.4; 2XSSC; 90% ethanol, and 90% ethanol (in that order, 7 minutes per solution.) The nitrocellulose filters were then baked _in vacuo_ at 80°C for 2 hours.

Prehybridization and hybridization procedures were as described above, except that the three washes were at room temperature. 90 positive hybridization signals were detected by autoradiography. Some of the "positive clones" were further analyzed by restriction analysis (e.g. _PstI_ digestion) and hybridization of "Southern blots" as above.

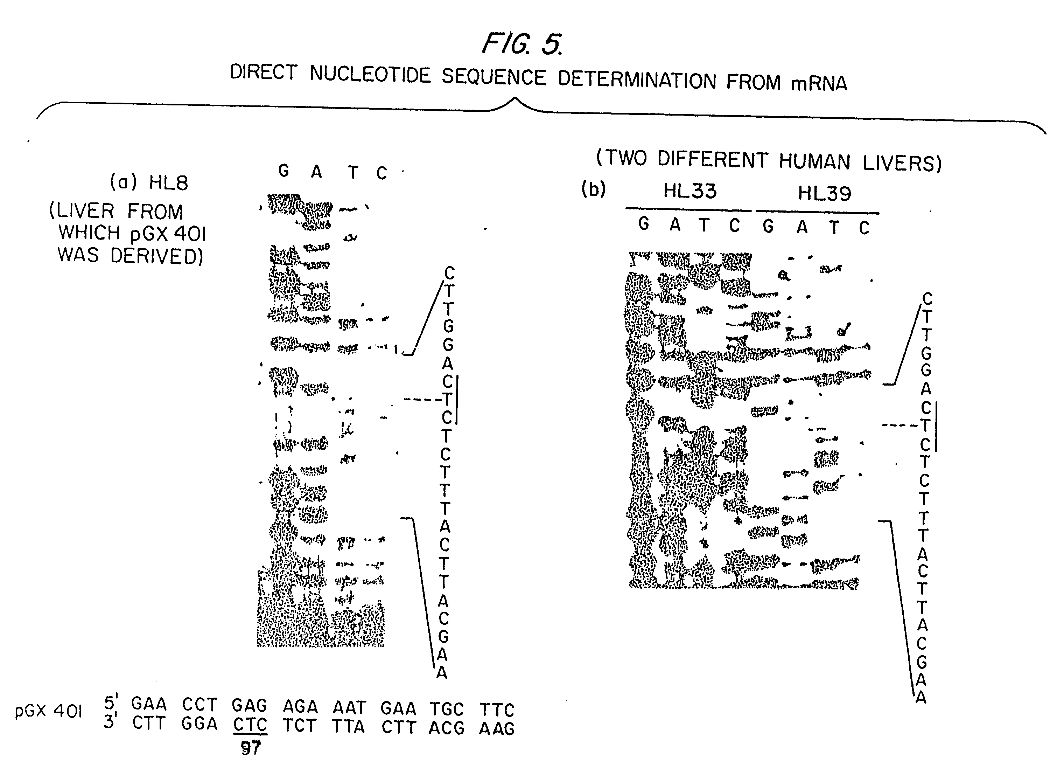

A clone bearing a full length HSA cDNA was identified and confirmed by DNA sequencing. The recombinant plasmid containing this HSA cDNA insert was termed pGX401 and is shown in figure 4. A partial restriction map of the HSA cDNA is shown in Figure 1, while Figure 2 shows the DNA sequence (5'→3' strand) of the cloned gene and the amino acid sequence it specifies.

A sample of _E. coli_ HB101 transformed with pGX401 has been deposited at the U.S. Dept. of Agriculture

24

Northern Regional Research Center in Peoria, Illinois, under accession number NRRL B-15784.

## EXAMPLE IV

### DNA Sequence Analysis of HSA cDNA Prepared from Human Liver Samples Taken from Different Individuals

In comparing the DNA sequence of the HSA cDNA insert in pGX401 (Example III) with the cDNA sequences published by Lawn et al., supra, and Dugaiczyk et al., supra, two codon differences were found that predict amino acid differences. The pGX401 sequence and the sequence reported by Lawn et al. indicated that codon 97 of the mature protein was GAG (glutamic acid), while Dugaiczyk et al. reported it to be GGG (glycine). In the pGX401 sequence and the sequence reported by Dugaiczyk codon 396 also was reported to be GAG (glutamic acid), and Lawn et al. reported that codon to be AAG (lysine).

To gain some insight into whether these differences represented true protein polymorphisms or merely experimental artifacts, the DNA sequence in the regions of codons 97 and 396 was determined for several new independent HSA genes.

Messenger RNA (mRNA) was isolated from normal human liver tissue taken from four different individuals. The procedures of Example I were followed except that sucrose gradient fractionation of oligo (dT)-cellulose-purified mRNA was omitted. Double stranded cDNA was synthesized from this mRNA template by the procedures described in Example II and poly(dC) "tails" were added according to Deng and Wu, NAR 9:4123, 1981.

The vector into which the dC-tailed cDNA was inserted was plasmid pGX1066. This plasmid comprises the phage $\lambda tR_1$ transcription terminator upstream of a bank of ten closely-spaced unique restriction sites, which in turn is upstream of the $\lambda 4S$ transcription terminator.

E. coli strain GX1170 [F' leu hsdR thi supE gal-1,2 lac xyl ara trpC9830 lacIq] transformed with pGX1066 has been deposited with the American Type Culture Collection, Rockville, Maryland, as ATCC No. 39955.

Plasmid pGX1066 was linearized with PstI and poly(dG) tails were added using the homopolymeric tailing method described by Deng and Wu (Nucleic Acids Res., 9: 4173 (1981)). The vector DNA and cDNA were then annealed as described in Example II. E. coli strain DH1 cells [F-, endA1, hsdR17 ($R_k$-, $M_k$-), supE44, thi1, λ⁻, recA1, gyrA96, relA1] were made competent and transformed with the annealing reaction mix. Both E. coli strain DH1 and the transformation procedure used are described by D. Hanahan (J. Molec. Biol., 166: 557 (1983)). Transformants were plated on LM plates (1% (w/v) Bacto tryptone, 0.5% (w/v) yeast extract, 10mM NaCl, 10mM $MgSO_4 \cdot 7H_2O$, 1.5% (w/v) Bacto agar) with 35µg/ml ampicillin added.

Transformed E. coli colonies were screened for the presence of HSA sequences by Grunstein-Hogness filter hybridization (Gergen et al., 1979, Nuc. Acids. Res. 7:2115; Wallace et al., 1981, Nuc. Acids Res. 9:879) using kinased oligomers or nick-translated HSA cDNA fragments as probes. For identification of clones carrying HSA cDNA containing codon 396, a synthetic oligonucleotide, 5' TTGTACTCTCCAAGCTGC 3', corresponding to codons 397-402 (and the last nucleotide of codon 396) was used. For detection of clones carrying HSA cDNA containing codon 97, either of two synthetic oligonucleotides, 5' TCTCTTCATTGTCATGAAAAGC 3', corresponding to codons 126-132 (and one nucleotide of codon 133), or 5' TTCTTGTTTTGCACAGC 3', corresponding to codons 90 (last 2 nucleotides) - 95, or a nick-translated HSA fragment (derived from pGX401), corresponding to codons -1 to 364 was used. Upon identification of clones containing the HSA sequence of interest, restriction

26

fragments were subcloned into an M13 phage. HSA cDNA-carrying phage were identified by screening plaques according to the procedure of Benton and Davis (Science, 196:180 (1977)). The DNA sequence was determined with these M13 clones by the dideoxy method (Biggin et al., Proc. Nat. Acad. Sci., U.S.A. 80:3963 (1983)).

By the procedures described above, transformants containing HSA cDNA that included codon 396 were derived from all four human livers. Transformants containing HSA cDNA that included codon 97 were derived from only two of the four livers. The DNA sequence in all cases (including 60 to 100 base pairs on each side of the codon in question) matched the sequence determined for pGX401.

Messenger RNA then was isolated from normal human liver samples taken from two more individuals, and the sequence at codon 97 was determined using a modification of the Sanger sequencing procedure in which reverse transcriptase was used to copy the single-stranded RNA template. A synthetic oligonucleotide, 5' TGTCTCTTCATTGTCATGAAAAGC 3', corresponding to codons 126-133, was used as a primer. The mRNA, purified by oligo (dT)-cellulose chromatography as previously described, was incubated in a reaction volume of 2µl containing 10 mM Tris · HCl (pH 8.3), 140 mM KCl, 10 mM MgCl$_2$, 20 mM β-mercaptoethanol, 1.6 mM dNTP, 0.2 mM ddNTP, 250 ng RNA, 5 ng kinased primer and 1.88 units reverse transcriptase (Life Sciences, Inc.). After overlaying the solution with 4 µl of mineral oil the reaction was incubated at 42°C for fifteen minutes and was terminated by the addition of 7 µl of 250 mM Na$_2$ EDTA. The mineral oil was extracted with ether and removed with a drawn-out pasteur pipette. Formamide loading buffer was added to the samples prior to electrophoresis on a urea sequencing gel. The gels were run until the bromphenol blue tracking dye had migrated to

27

the bottom. They then were dried under vacuum and exposed to X-ray film with two intensifying screens for periods between twelve hours and several days.

The HSA sequence at codon 97 for both liver samples was identical to the sequence at codon 97 in pGX401. (See Figure 5.) The reliability of the technique to determine nucleotide sequence from mRNA was evaluated using polyA$^+$ RNA prepared from the liver that was the source of the cDNA originally cloned in pGX401. The results (Figure 5) showed that the sequence determined in this manner was identical to the sequence originally determined in pGX401.

CLAIMS FOR THE DESIGNATED STATES: BE, DE, FR, IT,
LU, NL, SE, CH and UK

1.    A synthetic gene coding for human serum albumin.

2.    An isolated human serum albumin gene.

3.    An isolated prepro-human serum albumin gene.

4.    A human serum albumin gene as claimed in claim 1,
comprising the following deoxyribonucleotide sequence
which corresponds to the indicated amino acid sequence:

| Asp | Ala | His | Lys | Ser | Glu | Val | Ala |
|-----|-----|-----|-----|-----|-----|-----|-----|
| G A Y | G C X | C A Y | A A M | Q R S | G A M | G T X | G C X |

| His | Arg | Phe | Lys | Asp | Leu | Gly | Glu |
|-----|-----|-----|-----|-----|-----|-----|-----|
| C A Y | L G N | T T Y | A A M | G A Y | Y T Z | G G X | G A M |

| Glu | Asn | Phe | Lys | Ala | Leu | Val | Leu |
|-----|-----|-----|-----|-----|-----|-----|-----|
| G A M | A A Y | T T Y | A A M | G C X | Y T Z | G T X | Y T Z |

| Ile | Ala | Phe | Ala | Gln | Tyr | Leu | Gln |
|-----|-----|-----|-----|-----|-----|-----|-----|
| A T H | G C X | T T Y | G C X | C A M | T A Y | Y T Z | C A M |

| Gln | Cys | Pro | Phe | Glu | Asp | His | Val |
|-----|-----|-----|-----|-----|-----|-----|-----|
| C A M | T G Y | C C X | T T Y | G A M | G A Y | C A Y | G T X |

| Lys | Leu | Val | Asn | Glu | Val | Thr | Glu |
|-----|-----|-----|-----|-----|-----|-----|-----|
| A A M | Y T Z | G T X | A A Y | G A M | G T X | A C X | G A M |

| Phe | Ala | Lys | Thr | Cys | Val | Ala | Asp |
|-----|-----|-----|-----|-----|-----|-----|-----|
| T T Y | G C X | A A M | A C X | T G Y | G T X | G C X | G A Y |

| Glu | Ser | Ala | Glu | Asn | Cys | Asp | Lys |
|-----|-----|-----|-----|-----|-----|-----|-----|
| G A M | Q R S | G C X | G A M | A A Y | T G Y | G A Y | A A M |

| Ser | Leu | His | Thr | Leu | Phe | Gly | Asp |
|-----|-----|-----|-----|-----|-----|-----|-----|
| Q R S | Y T Z | C A Y | A C X | Y T Z | T T Y | G G X | G A Y |

| Lys | Leu | Cys | Thr | Val | Ala | Thr | Leu |
|-----|-----|-----|-----|-----|-----|-----|-----|
| A A M | Y T Z | T G Y | A C X | G T X | G C X | A C X | Y T Z |

| Arg | Glu | Thr | Tyr | Gly | Glu | Met | Ala |
|-----|-----|-----|-----|-----|-----|-----|-----|
| L G N | G A M | A C X | T A Y | G G X | G A M | A T G | G C X |

| Asp | Cys | Cys | Ala | Lys | Gln | Glu | Pro |
|-----|-----|-----|-----|-----|-----|-----|-----|
| G A Y | T G Y | T G Y | G C X | A A M | C A M | G A M | C C X |

```
Glu     Arg     Asn     Glu     Cys     Phe     Leu     Gln
G A M   L G N   A A Y   G A M   T G Y   T T Y   Y T Z   C A M

His     Lys     Asp     Asp     Asn     Pro     Asn     Leu
C A Y   A A M   G A Y   G A Y   A A Y   C C X   A A Y   Y T Z

Pro     Arg     Leu     Val     Arg     Pro     Glu     Val
C C X   L G N   Y T Z   G T X   L G N   C C X   G A M   G T X

Asp     Val     Met     Cys     Thr     Ala     Phe     His
G A Y   G T X   A T G   T G Y   A C X   G C X   T T Y   C A Y

Asp     Asn     Glu     Glu     Thr     Phe     Leu     Lys
G A Y   A A Y   G A M   G A M   A C X   T T Y   Y T Z   A A M

Lys     Tyr     Leu     Tyr     Glu     Ile     Ala     Arg
A A M   T A Y   Y T Z   T A Y   G A M   A T H   G C X   L G N

Arg     His     Pro     Tyr     Phe     Thr     Ala     Pro
L G N   C A Y   C C X   T A Y   T T Y   A C X   G C X   C C X

Glu     Leu     Leu     Phe     Phe     Ala     Lys     Arg
G A M   Y T Z   Y T Z   T T Y   T T Y   G C X   A A M   L G N

Tyr     Lys     Ala     Ala     Phe     Thr     Glu     Cys
T A Y   A A M   G C X   G C X   T T Y   A C X   G A M   T G Y

Cys     Ala     Gln     Ala     Asp     Lys     Ala     Ala
T G Y   G C X   C A M   G C X   G A Y   A A M   G C X   G C X

Cys     Leu     Phe     Pro     Lys     Leu     Asp     Glu
T G Y   Y T Z   T T Y   C C X   A A M   Y T Z   G A Y   G A M

Leu     Arg     Asp     Glu     Gly     Lys     Ala     Ser
Y T Z   L G N   G A Y   G A M   G G X   A A M   G C X   Q R S

Ser     Ala     Lys     Gln     Arg     Leu     Lys     Cys
Q R S   G C X   A A M   C A M   L G N   Y T Z   A A M   T G Y

Ala     Ser     Leu     Gln     Lys     Phe     Gly     Glu
G C X   Q R S   Y T Z   C A M   A A M   T T Y   G G X   G A M

Arg     Ala     Phe     Lys     Ala     Trp     Ala     Val
L G N   G C X   T T Y   A A M   G C X   T G G   G C X   G T X

Ala     Arg     Leu     Ser     Gln     Arg     Phe     Pro
G C X   L G N   Y T Z   Q R S   C A M   L G N   T T Y   C C X

Lys     Ala     Glu     Phe     Ala     Glu     Val     Ser
A A M   G C X   G A M   T T Y   G C X   G A M   G T X   Q R S

Lys     Phe     Val     Thr     Asp     Leu     Thr     Lys
A A M   T T Y   G T X   A C X   G A Y   Y T Z   A C X   A A M

Val     His     Thr     Glu     Cys     Cys     His     Gly
G T X   C A Y   A C X   G A M   T G Y   T G Y   C A Y   G G X
```

30

| Asp | Leu | Leu | Glu | Cys | Ala | Asp | Asp |
|-----|-----|-----|-----|-----|-----|-----|-----|
| G A Y | Y T Z | Y T Z | G A M | T G Y | G C X | G A Y | G A Y |

| Arg | Ala | Asp | Leu | Ala | Lys | Tyr | Ile |
|-----|-----|-----|-----|-----|-----|-----|-----|
| L G N | G C X | G A Y | Y T Z | G C X | A A M | T A Y | A T H |

| Cys | Glu | Asn | Gln | Asp | Ser | Ile | Ser |
|-----|-----|-----|-----|-----|-----|-----|-----|
| T G Y | G A M | A A Y | C A M | G A Y | Q R S | A T H | Q R S |

| Ser | Lys | Leu | Lys | Glu | Cys | Cys | Glu |
|-----|-----|-----|-----|-----|-----|-----|-----|
| Q R S | A A M | Y T Z | A A M | G A M | T G Y | T G Y | G A M |

| Lys | Pro | Leu | Phe | Glu | Lys | Ser | His |
|-----|-----|-----|-----|-----|-----|-----|-----|
| A A M | C C X | Y T Z | T T Y | G A M | A A M | Q R S | C A Y |

| Cys | Ile | Ala | Glu | Val | Glu | Asn | Asp |
|-----|-----|-----|-----|-----|-----|-----|-----|
| T G Y | A T H | G C X | G A M | G T X | G A M | A A Y | G A Y |

| Glu | Met | Pro | Ala | Asp | Phe | Pro | Ser |
|-----|-----|-----|-----|-----|-----|-----|-----|
| G A M | A T G | C C X | G C X | G A Y | T T Y | C C X | Q R S |

| Phe | Ala | Val | Asp | Phe | Val | Glu | Ser |
|-----|-----|-----|-----|-----|-----|-----|-----|
| T T Y | G C X | G T X | G A Y | T T Y | G T X | G A M | Q R S |

| Lys | Asp | Val | Cys | Lys | Asn | Tyr | Ala |
|-----|-----|-----|-----|-----|-----|-----|-----|
| A A M | G A Y | G T X | T G Y | A A M | A A Y | T A Y | G C X |

| Glu | Ala | Lys | Asp | Val | Phe | Leu | Gly |
|-----|-----|-----|-----|-----|-----|-----|-----|
| G A M | G C X | A A M | G A Y | G T X | T T Y | Y T Z | G G X |

| Met | Phe | Phe | Tyr | Glu | Tyr | Ala | Arg |
|-----|-----|-----|-----|-----|-----|-----|-----|
| A T G | T T Y | T T Y | T A Y | G A M | T A Y | G C X | L G N |

| Arg | His | Pro | Asp | Tyr | Ser | Val | Val |
|-----|-----|-----|-----|-----|-----|-----|-----|
| L G N | C A Y | C C X | G A Y | T A Y | Q R S | G T X | G T X |

| Leu | Leu | Leu | Arg | Leu | Ala | Lys | Thr |
|-----|-----|-----|-----|-----|-----|-----|-----|
| Y T Z | Y T Z | Y T Z | L G N | Y T Z | G C X | A A M | A C X |

| Tyr | Glu | Thr | Thr | Leu | Glu | Lys | Cys |
|-----|-----|-----|-----|-----|-----|-----|-----|
| T A Y | G A M | A C X | A C X | Y T Z | G A M | A A M | T G Y |

| Cys | Ala | Ala | Ala | Asp | Pro | His | Glu |
|-----|-----|-----|-----|-----|-----|-----|-----|
| T G Y | G C X | G C X | G C X | G A Y | C C X | C A Y | G A M |

| Cys | Tyr | Ala | Lys | Val | Phe | Asp | Glu |
|-----|-----|-----|-----|-----|-----|-----|-----|
| T G Y | T A Y | G C X | A A M | G T X | T T Y | G A Y | G A M |

| Phe | Lys | Pro | Pro | Val | Glu | Glu | Pro |
|-----|-----|-----|-----|-----|-----|-----|-----|
| T T Y | A A M | C C X | C C X | G T X | G A M | G A M | C C X |

```
Gln     Asn     Phe     Ile     Lys     Gln     Asn     Cys
C A M   A A Y   T T Y   A T H   A A M   C A M   A A Y   T G Y

Glu     Leu     Phe     Glu     Gln     Leu     Gly     Glu
G A M   Y T Z   T T Y   G A M   C A M   Y T Z   G G X   G A M

Tyr     Lys     Phe     Gln     Asn     Ala     Leu     Phe
T A Y   A A M   T T Y   C A M   A A Y   G C X   Y T Z   T T Y

Val     Arg     Tyr     Thr     Lys     Lys     Val     Pro
G T X   L G N   T A Y   A C X   A A M   A A M   G T X   C C X

Gln     Leu     Ser     Thr     Pro     Thr     Leu     Val
C A M   Y T Z   Q R S   A C X   C C X   A C X   Y T Z   G T X

Glu     Val     Ser     Arg     Asn     Leu     Gly     Lys
G A M   G T X   Q R S   L G N   A A Y   Y T Z   G G X   A A M

Val     Gly     Ser     Lys     Cys     Cys     Lys     His
G T X   G G X   Q R S   A A M   T G Y   T G Y   A A M   C A Y

Pro     Glu     Ala     Lys     Arg     Met     Pro     Cys
C C X   G A M   G C X   A A M   L G N   A T G   C C X   T G Y

Ala     Glu     Asp     Tyr     Leu     Ser     Val     Val
G C X   G A M   G A Y   T A Y   Y T Z   Q R S   G T X   G T X

Leu     Asn     Gln     Leu     Cys     Val     Leu     His
Y T Z   A A Y   C A M   Y T Z   T G Y   G T X   Y T Z   C A Y

Glu     Lys     Thr     Pro     Val     Ser     Asp     Arg
G A M   A A M   A C X   C C X   G T X   Q R S   G A Y   L G N

Val     Thr     Lys     Cys     Cys     Thr     Glu     Ser
G T X   A C X   A A M   T G Y   T G Y   A C X   G A M   Q R S

Leu     Val     Asn     Arg     Arg     Pro     Gly     Phe
Y T Z   G T X   A A Y   L G N   L G N   C C X   G G X   T T Y

Ser     Ala     Leu     Glu     Val     Asp     Glu     Thr
Q R S   G C X   Y T Z   G A M   G T X   G A Y   G A M   A C X

Tyr     Val     Pro     Lys     Glu     Phe     Asn     Ala
T A Y   G T X   C C X   A A M   G A M   T T Y   A A Y   G C X

Glu     Thr     Phe     Thr     Phe     His     Ala     Asp
G A M   A C X   T T Y   A C X   T T Y   C A Y   G C X   G A Y

Ile     Cys     Thr     Leu     Ser     Glu     Lys     Glu
A T H   T G Y   A C X   Y T Z   Q R S   G A M   A A M   G A M
```

32

```
Arg     Gln     Ile     Lys     Lys     Glu     Thr     Ala
L G N   C A M   A T H   A A M   A A M   G A M   A C X   G C X

Leu     Val     Glu     Leu     Val     Lys     His     Lys
Y T Z   G T X   G A M   Y T Z   G T X   A A M   C A Y   A A M

Pro     Lys     Ala     Thr     Lys     Glu     Glu     Leu
C C X   A A M   G C X   A C X   A A M   G A M   G A M   Y T Z

Lys     Ala     Val     Met     Asp     Asp     Phe     Ala
A A M   G C X   G T X   A T G   G A Y   G A Y   T T Y   G C X

Ala     Phe     Val     Glu     Lys     Cys     Cys     Lys
G C X   T T Y   G T X   G A M   A A M   T G Y   T G Y   A A M

Ala     Asp     Asp     Lys     Glu     Thr     Cys     Phe
G C X   G A Y   G A Y   A A M   G A M   A C X   T G Y   T T Y

Ala     Glu     Glu     Gly     Lys     Lys     Leu     Val
G C X   G A M   G A M   G G X   A A M   A A M   Y T Z   G T X

Ala     Ala     Ser     Glu     Ala     Val     Leu     Gly
G C X   G C X   Q R S   G A M   G C X   G T X   Y T Z   G G X

Leu
Y T Z   T A A
```

wherein, the 5' to 3' strand, beginning with the amino terminus and the amino acids for which each triplet codes are shown, and wherein the abbreviations have the following standard meanings:

A is deoxyadenyl

T is thymidyl

G is deoxyguanyl

C is deoxycytosyl

X is A, T, C or G

Y is T or C

When Y is C, Z is A, T, C or G

When Y is T, Z is A or G

H is A, T or C

Q is T or A

When Q is T, R is C and S is A, T, C or G

When Q is A, R is G and S is T or C

M is A or G

L is A or C

When L is A, N is A or G

When L is C, N is A, T, C or G

GLY is glycine

ALA is alanine

VAL is valine

LEU is leucine

ILE is isoleucine

SER is serine

THR is threonine

PHE is phenylalanine

TYR is tyrosine

TRP is tyryptophan

CYS is cysteine

MET is methionine

ASP is aspartic acid

GLU is glutamic acid

LYS is lysine

ARG is arginine

HIS is histidine

PRO is proline

GLN is glutamine

ASN is asparagine

5.    A prepro-serum albumin gene as claimed in claim 1
comprising the following deoxyribonucleotide sequence:

| Met | Lys | Trp | Val | Thr | Phe | |
|-----|-----|-----|-----|-----|-----|-----|
| A T G | A A M | T G G | G T X | A C X | T T Y | |
| Ile | Ser | Leu | Leu | Phe | Leu | Phe |
| A T H | Q R S | Y T Z | Y T Z | T T Y | Y T Z | T T Y |
| Ser | Ser | Ala | Tyr | Ser | Arg | Gly |
| Q R S | Q R S | G C X | T A Y | Q R S | L G N | G G X |
| Val | Phe | Arg | Arg | Asp | Ala | His | Lys |
| G T X | T T Y | L G N | L G N | G A Y | G C X | C A Y | A A M |

34

```
Ser     Glu     Val     Ala     His     Arg     Phe     Lys
Q R S   G A M   G T X   G C X   C A Y   L G N   T T Y   A A M

Asp     Leu     Gly     Glu     Glu     Asn     Phe     Lys
G A Y   Y T Z   G G X   G A M   G A M   A A Y   T T Y   A A M

Ala     Leu     Val     Leu     Ile     Ala     Phe     Ala
G C X   Y T Z   G T X   Y T Z   A T H   G C X   T T Y   G C X

Gln     Tyr     Leu     Gln     Gln     Cys     Pro     Phe
C A M   T A Y   Y T Z   C A M   C A M   T G Y   C C X   T T Y

Glu     Asp     His     Val     Lys     Leu     Val     Asn
G A M   G A Y   C A Y   G T X   A A M   Y T Z   G T X   A A Y

Glu     Val     Thr     Glu     Phe     Ala     Lys     Thr
G A M   G T X   A C X   G A M   T T Y   G C X   A A M   A C X

Cys     Val     Ala     Asp     Glu     Ser     Ala     Glu
T G Y   G T X   G C X   G A Y   G A M   Q R S   G C X   G A M

Asn     Cys     Asp     Lys     Ser     Leu     His     Thr
A A Y   T G Y   G A Y   A A M   Q R S   Y T Z   C A Y   A C X

Leu     Phe     Gly     Asp     Lys     Leu     Cys     Thr
Y T Z   T T Y   G G X   G A Y   A A M   Y T Z   T G Y   A C X

Val     Ala     Thr     Leu     Arg     Glu     Thr     Tyr
G T X   G C X   A C X   Y T Z   L G N   G A M   A C X   T A Y

Gly     Glu     Met     Ala     Asp     Cys     Cys     Ala
G G X   G A M   A T G   G C X   G A Y   T G Y   T G Y   G C X

Lys     Gln     Glu     Pro     Glu     Arg     Asn     Glu
A A M   C A M   G A M   C C X   G A M   L G N   A A Y   G A M

Cys     Phe     Leu     Gln     His     Lys     Asp     Asp
T G Y   T T Y   Y T Z   C A M   C A Y   A A M   G A Y   G A Y

Asn     Pro     Asn     Leu     Pro     Arg     Leu     Val
A A Y   C C X   A A Y   Y T Z   C C X   L G N   Y T Z   G T X

Arg     Pro     Glu     Val     Asp     Val     Met     Cys
L G N   C C X   G A M   G T X   G A Y   G T X   A T G   T G Y

Thr     Ala     Phe     His     Asp     Asn     Glu     Glu
A C X   G C X   T T Y   C A Y   G A Y   A A Y   G A M   G A M

Thr     Phe     Leu     Lys     Lys     Tyr     Leu     Tyr
A C X   T T Y   Y T Z   A A M   A A M   T A Y   Y T Z   T A Y
```

```
Glu     Ile     Ala     Arg     Arg     His     Pro     Tyr
G A M   A T H   G C X   L G N   L G N   C A Y   C C X   T A Y

Phe     Thr     Ala     Pro     Glu     Leu     Leu     Phe
T T Y   A C X   G C X   C C X   G A M   Y T Z   Y T Z   T T Y

Phe     Ala     Lys     Arg     Tyr     Lys     Ala     Ala
T T Y   G C X   A A M   L G N   T A Y   A A M   G C X   G C X

Phe     Thr     Glu     Cys     Cys     Ala     Gln     Ala
T T Y   A C X   G A M   T G Y   T G Y   G C X   C A M   G C X

Asp     Lys     Ala     Ala     Cys     Leu     Phe     Pro
G A Y   A A M   G C X   G C X   T G Y   Y T Z   T T Y   C C X

Lys     Leu     Asp     Glu     Leu     Arg     Asp     Glu
A A M   Y T Z   G A Y   G A M   Y T Z   L G N   G A Y   G A M

Gly     Lys     Ala     Ser     Ser     Ala     Lys     Gln
G G X   A A M   G C X   Q R S   Q R S   G C X   A A M   C A M

Arg     Leu     Lys     Cys     Ala     Ser     Leu     Gln
L G N   Y T Z   A A M   T G Y   G C X   Q R S   Y T Z   C A M

Lys     Phe     Gly     Glu     Arg     Ala     Phe     Lys
A A M   T T Y   G G X   G A M   L G N   G C X   T T Y   A A M

Ala     Trp     Ala     Val     Ala     Arg     Leu     Ser
G C X   T G G   G C X   G T X   G C X   L G N   Y T Z   Q R S

Gln     Arg     Phe     Pro     Lys     Ala     Glu     Phe
C A M   L G N   T T Y   C C X   A A M   G C X   G A M   T T Y

Ala     Glu     Val     Ser     Lys     Phe     Val     Thr
G C X   G A M   G T X   Q R S   A A M   T T Y   G T X   A C X

Asp     Leu     Thr     Lys     Val     His     Thr     Glu
G A Y   Y T Z   A C X   A A M   G T X   C A Y   A C X   G A M

Cys     Cys     His     Gly     Asp     Leu     Leu     Glu
T G Y   T G Y   C A Y   G G X   G A Y   Y T Z   Y T Z   G A M

Cys     Ala     Asp     Asp     Arg     Ala     Asp     Leu
T G Y   G C X   G A Y   G A Y   L G N   G C X   G A Y   Y T Z

Ala     Lys     Tyr     Ile     Cys     Glu     Asn     Gln
G C X   A A M   T A Y   A T H   T G Y   G A M   A A Y   C A M

Asp     Ser     Ile     Ser     Ser     Lys     Leu     Lys
G A Y   Q R S   A T H   Q R S   Q R S   A A M   Y T Z   A A M
```

36

```
Glu     Cys     Cys     Glu     Lys     Pro     Leu     Phe
G A M   T G Y   T G Y   G A M   A A M   C C X   Y T Z   T T Y

Glu     Lys     Ser     His     Cys     Ile     Ala     Glu
G A M   A A M   Q R S   C A Y   T G Y   A T H   G C X   G A M

Val     Glu     Asn     Asp     Glu     Met     Pro     Ala
G T X   G A M   A A Y   G A Y   G A M   A T G   C C X   G C X

Asp     Phe     Pro     Ser     Phe     Ala     Val     Asp
G A Y   T T Y   C C X   Q R S   T T Y   G C X   G T X   G A Y

Phe     Val     Glu     Ser     Lys     Asp     Val     Cys
T T Y   G T X   G A M   Q R S   A A M   G A Y   G T X   T G Y

Lys     Asn     Tyr     Ala     Glu     Ala     Lys     Asp
A A M   A A Y   T A Y   G C X   G A M   G C X   A A M   G A Y

Val     Phe     Leu     Gly     Met     Phe     Phe     Tyr
G T X   T T Y   Y T Z   G G X   A T G   T T Y   T T Y   T A Y

Glu     Tyr     Ala     Arg     Arg     His     Pro     Asp
G A M   T A Y   G C X   L G N   L G N   C A Y   C C X   G A Y

Tyr     Ser     Val     Val     Leu     Leu     Leu     Arg
T A Y   Q R S   G T X   G T X   Y T Z   Y T Z   Y T Z   L G N

Leu     Ala     Lys     Thr     Tyr     Glu     Thr     Thr
Y T Z   G C X   A A M   A C X   T A Y   G A M   A C X   A C X

Leu     Glu     Lys     Cys     Cys     Ala     Ala     Ala
Y T Z   G A M   A A M   T G Y   T G Y   G C X   G C X   G C X

Asp     Pro     His     Glu     Cys     Tyr     Ala     Lys
G A Y   C C X   C A Y   G A M   T G Y   T A Y   G C X   A A M

Val     Phe     Asp     Glu     Phe     Lys     Pro     Pro
G T X   T T Y   G A Y   G A M   T T Y   A A M   C C X   C C X

Val     Glu     Glu     Pro     Gln     Asn     Phe     Ile
G T X   G A M   G A M   C C X   C A M   A A Y   T T Y   A T H

Lys     Gln     Asn     Cys     Glu     Leu     Phe     Glu
A A M   C A M   A A Y   T G Y   G A M   Y T Z   T T Y   G A M

Gln     Leu     Gly     Glu     Tyr     Lys     Phe     Gln
C A M   Y T Z   G G X   G A M   T A Y   A A M   T T Y   C A M

Asn     Ala     Leu     Phe     Val     Arg     Tyr     Thr
A A Y   G C X   Y T Z   T T Y   G T X   L G N   T A Y   A C X
```

```
Lys     Lys     Val     Pro     Gln     Leu     Ser     Thr
A A M   A A M   G T X   C C X   C A M   Y T Z   Q R S   A C X

Pro     Thr     Leu     Val     Glu     Val     Ser     Arg
C C X   A C X   Y T Z   G T X   G A M   G T X   Q R S   L G N

Asn     Leu     Gly     Lys     Val     Gly     Ser     Lys
A A Y   Y T Z   G G X   A A M   G T X   G G X   Q R S   A A M

Cys     Cys     Lys     His     Pro     Glu     Ala     Lys
T G Y   T G Y   A A M   C A Y   C C X   G A M   G C X   A A M

Arg     Met     Pro     Cys     Ala     Glu     Asp     Tyr
L G N   A T G   C C X   T G Y   G C X   G A M   G A Y   T A Y

Leu     Ser     Val     Val     Leu     Asn     Gln     Leu
Y T Z   Q R S   G T X   G T X   Y T Z   A A Y   C A M   Y T Z

Cys     Val     Leu     His     Glu     Lys     Thr     Pro
T G Y   G T X   Y T Z   C A Y   G A M   A A M   A C X   C C X

Val     Ser     Asp     Arg     Val     Thr     Lys     Cys
G T X   Q R S   G A Y   L G N   G T X   A C X   A A M   T G Y

Cys     Thr     Glu     Ser     Leu     Val     Asn     Arg
T G Y   A C X   G A M   Q R S   Y T Z   G T X   A A Y   L G N

Arg     Pro     Gly     Phe     Ser     Ala     Leu     Glu
L G N   C C X   G G X   T T Y   Q R S   G C X   Y T Z   G A M

Val     Asp     Glu     Thr     Tyr     Val     Pro     Lys
G T X   G A Y   G A M   A C X   T A Y   G T X   C C X   A A M

Glu     Phe     Asn     Ala     Glu     Thr     Phe     Thr
G A M   T T Y   A A Y   G C X   G A M   A C X   T T Y   A C X

Phe     His     Ala     Asp     Ile     Cys     Thr     Leu
T T Y   C A Y   G C X   G A Y   A T H   T G Y   A C X   Y T Z

Ser     Glu     Lys     Glu     Arg     Gln     Ile     Lys
Q R S   G A M   A A M   G A M   L G N   C A M   A T H   A A M

Lys     Glu     Thr     Ala     Leu     Val     Glu     Leu
A A M   G A M   A C X   G C X   Y T Z   G T X   G A M   Y T Z

Val     Lys     His     Lys     Pro     Lys     Ala     Thr
G T X   A A M   C A Y   A A M   C C X   A A M   G C X   A C X

Lys     Glu     Glu     Leu     Lys     Ala     Val     Met
A A M   G A M   G A M   Y T Z   A A M   G C X   G T X   A T G

Asp     Asp     Phe     Ala     Ala     Phe     Val     Glu
G A Y   G A Y   T T Y   G C X   G C X   T T Y   G T X   G A M
```

```
Lys      Cys      Cys      Lys      Ala      Asp      Asp      Lys
A A M    T G Y    T G Y    A A M    G C X    G A Y    G A Y    A A M

Glu      Thr      Cys      Phe      Ala      Glu      Glu      Gly
G A M    A C X    T G Y    T T Y    G C X    G A M    G A M    G G X

Lys      Lys      Leu      Val      Ala      Ala      Ser      Glu
A A M    A A M    Y T Z    G T X    G C X    G C X    Q R S    G A M

Ala      Val      Leu      Gly      Leu
G C X    G T X    Y T Z    G G X    Y T Z    T A A
```

wherein the 5' and 3' strand, beginning with the amino
terminus, and the amino acids for which each triplet
codes are shown, and wherein the abbreviations are
defined as in claim 4.

6.    A human serum albumin gene as claimed in claim 4
comprising the following deoxyribonucleotide sequence:

```
GAT GCA CAC AAG AGT GAG GTT GCT CAT CGG TTT AAA GAT TTG
GGA GAA GAA AAT TTC AAA GCC TTG GTG TTG ATT GCC TTT GCT
CAG TAT CTT CAG CAG TGT CCA TTT GAA GAT CAT GTA AAA TTA
GTG AAT GAA GTA ACT GAA TTT GCA AAA ACA TGT GTT GCT GAT
GAG TCA GCT GAA AAT TGT GAC AAA TCA CTT CAT ACC CTT TTT
GGA GAC AAA TTA TGC ACA GTT GCA ACT CTT CGT GAA ACC TAT
GGT GAA ATG GCT GAC TGC TGT GCA AAA CAA GAA CCT GAG AGA
AAT GAA TGC TTC TTG CAA CAC AAA GAT GAC AAC CCA AAC CTC
CCC CGA TTG GTG AGA CCA GAG GTT GAT GTG ATG TGC ACT GCT
TTT CAT GAC AAT GAA GAG ACA TTT TTG AAA AAA TAC TTA TAT
GAA ATT GCC AGA AGA CAT CCT TAC TTT TAT GCC CCG GAA CTC
CTT TTC TTT GCT AAA AGG TAT AAA GCT GCT TTT ACA GAA TGT
TGC CAA GCT GCT GAT AAA GCT GCC TGC CTG TTG CCA AAG CTC
GAT GAA CTT CGG GAT GAA GGG AAG GCT TCG TCT GCC AAA CAG
AGA CTC AAG TGT GCC AGT CTC CAA AAA TTT GGA GAA AGA GCT
TTC AAA GCA TGG GCG GTG GCT CGC CTG AGC CAG AGA TTT CCC
AAA GCT GAG TTT GCA GAA GTT TCC AAG TTA GTG ACA GAT CTT
ACC AAA GTC CAC ACG GAA TGC TGC CAT GGA GAT CTG CTT GAA
TGT GCT GAT GAC AGG GCG GAC CTT GCC AAG TAT ATC TGT GAA
AAT CAA GAT TCG ATC TCC AGT AAA CTG AAG GAA TGC TGT GAA
```

```
AAA CCT CTG TTG GAA AAA TCC CAC TGC ATT GCC GAA GTG GAA
AAT GAT GAG ATG CCT GCT GAC TTG CCT TCA TTA GCT GCT GAT
TTT GTT GAA AGT AAG GAT GTT TGC AAA AAC TAT GCT GAG GCA
AAG GAT GTC TTC CTG GGC ATG TTT TTG TAT GAA TAT GCA AGA
AGG CAT CCT GAT TAC TCT GTC GTG CTG CTG CTG AGA CTT GCC
AAG ACA TAT GAA ACC ACT CTA GAG AAG TGC TGT GCC GCT GCA
GAT CCT CAT GAA TGC TAT GCC AAA GTG TTC GAT GAA TTT AAA
CCT CCT GTG GAA GAG CCT CAG AAT TTA ATC AAA CAA AAT TGT
GAG CTT TTT GAG CAG CTT GGA GAG TAC AAA TTC CAG AAT GCG
CTA TTA GTT CGT TAC ACC AAG AAA GTA CCC CAA GTG TCA ACT
CCA ACT CTT GTA GAG GTC TCA AGA AAC CTA GGA AAA GTG GGC
AGC AAA TGT TGT AAA CAT CCT GAA GCA AAA AGA ATG CCC TGT
GCA GAA GAC TAT CTA TCC GTG GTC CTG AAC CAG TTA TGT GTG
TTG CAT GAG AAA ACG CCA GTA AGT GAC AGA GTC ACC AAA TGC
TGC ACA GAA TCC TTG GTG AAC AGG CGA CCA TGC TTT TCA GCT
CTG GAA GTC GAT GAA ACA TAC GTT CCC AAA GAG TTT AAT GCT
GAA ACA TTC ACC TTC CAT GCA GAT ATA TGC ACA CTT TCT GAG
AAG GAG AGA CAA ATC AAG AAA CAA ACT GCA CTT GTT GAG CTC
GTG AAA CAC AAG CCC AAG GCA ACA AAA GAG CAA CTG AAA GCT
GTT ATG GAT GAT TTC GCA GCT TTT GTA GAG AAG TGC TGC AAG
GCT GAC GAT AAG GAG ACC TGC TTT GCC GAG GAG GGT AAA AAA
CTT GTT GCT GCA AGT CAA GCT GCC TTA GGC TTA TAA
```
wherein the 5' to 3' strand, beginning with the amino
terminus is shown, and wherein the abbreviations are
defined as in claim 4.

7.    A human prepro-serum albumin gene as claimed in claim 5
comprising the following deoxyribonucleotide sequence:

```
ATG AAG TGG GTA ACC TTT ATT TCC CTT CTT TTT CTC TTT AGC
TCG GCT TAT TCC AGG GGT GTG TTT CGT CGA GAT GCA CAC AAG
AGT GAG GTT GCT CAT CGG TTT AAA GAT TTG GGA GAA GAA AAT
TTC AAA GCC TTG GTG TTG ATT GCC TTT GCT CAG TAT CTT CAG
CAG TGT CCA TTT GAA GAT CAT GTA AAA TTA GTG AAT GAA GTA
ACT GAA TTT GCA AAA ACA TGT GTT GCT GAT GAG TCA GCT GAA
```

0206733

40

AAT TGT GAC AAA TCA CTT CAT ACC CTT TTT GGA GAC AAA TTA
TGC ACA GTT GCA ACT CTT CGT GAA ACC TAT GGT GAA ATG GCT
GAC TGC TGT GCA AAA CAA GAA CCT GAG AGA AAT GAA TGC TTC
TTG CAA CAC AAA GAT GAC AAC CCA AAC CTC CCC CGA TTG GTG
AGA CCA GAG GTT GAT GTG ATG TGC ACT GCT TTT CAT GAC AAT
GAA GAG ACA TTT TTG AAA AAA TAC TTA TAT GAA ATT GCC AGA
AGA CAT CCT TAC TTT TAT GCC CCG GAA CTC CTT TTC TTT GCT
AAA AGG TAT AAA GCT GCT TTT ACA GAA TGT TGC CAA GCT GCT
GAT AAA GCT GCC TGC CTG TTG CCA AAG CTC GAT GAA CTT CGG
GAT GAA GGG AAG GCT TCG TCT GCC AAA CAG AGA CTC AAG TGT
GCC AGT CTC CAA AAA TTT GGA GAA AGA GCT TTC AAA GCA TGG
GCG GTG GCT CGC CTG AGC CAG AGA TTT CCC AAA GCT GAG TTT
GCA GAA GTT TCC AAG TTA GTG ACA GAT CTT ACC AAA GTC CAC
ACG GAA TGC TGC CAT GGA GAT CTG CTT GAA TGT GCT GAT GAC
AGG GCG GAC CTT GCC AAG TAT ATC TGT GAA AAT CAA GAT TCG
ATC TCC AGT AAA CTG AAG GAA TGC TGT GAA AAA CCT CTG TTG
GAA AAA TCC CAC TGC ATT GCC GAA GTG GAA AAT GAT GAG ATG
CCT GCT GAC TTG CCT TCA TTA GCT GCT GAT TTT GTT GAA AGT
AAG GAT GTT TGC AAA AAC TAT GCT GAG GCA AAG GAT GTC TTC
CTG GGC ATG TTT TTG TAT GAA TAT GCA AGA AGG CAT CCT GAT
TAC TCT GTC GTG CTG CTG CTG AGA CTT GCC AAG ACA TAT GAA
ACC ACT CTA GAG AAG TGC TGT GCC GCT GCA GAT CCT CAT GAA
TGC TAT GCC AAA GTG TTC GAT GAA TTT AAA CCT CCT GTG GAA
GAG CCT CAG AAT TTA ATC AAA CAA AAT TGT GAG CTT TTT GAG
CAG CTT GGA GAG TAC AAA TTC CAG AAT GCG CTA TTA GTT CGT
TAC ACC AAG AAA GTA CCC CAA GTG TCA ACT CCA ACT CTT GTA
GAG GTC TCA AGA AAC CTA GGA AAA GTG GGC AGC AAA TGT TGT
AAA CAT CCT GAA GCA AAA AGA ATG CCC TGT GCA GAA GAC TAT
CTA TCC GTG GTC CTG AAC CAG TTA TGT GTG TTG CAT GAG AAA
ACG CCA GTA AGT GAC AGA GTC ACC AAA TGC TGC ACA GAA TCC
TTG GTG AAC AGG CGA CCA TGC TTT TCA GCT CTG GAA GTC GAT
GAA ACA TAC GTT CCC AAA GAG TTT AAT GCT GAA ACA TTC ACC
TTC CAT GCA GAT ATA TGC ACA CTT TCT GAG AAG GAG AGA CAA
ATC AAG AAA CAA ACT GCA CTT GTT GAG CTC GTG AAA CAC AAG
CCC AAG GCA ACA AAA GAG CAA CTG AAA GCT GTT ATG GAT GAT

41

TTC GCA GCT TTT GTA GAG AAG TGC TGC AAG GCT GAC GAT AAG
GAG ACC TGC TTT GCC GAG GAG GGT AAA AAA CTT GTT GCT GCA
AGT CAA GCT GCC TTA GGC TTA TAA
wherein the 5' to 3' strand, beginning with the amino
terminus is shown, and wherein the abbreviations are
defined as in claim 4.

8.      A human prepro-serum albumin gene as claimed in claim 7
comprised in the following deoxyribonucleotide sequence:
5'

    TCTCTTCTGTCAACCCCACGCCTTTGGCACA ATG AAG TGG GTA
ACC TTT ATT TCC CTT CTT TTT CTC TTT AGC TCG GCT TAT TCC
AGG GGT GTG TTT CGT CGA GAT GCA CAC AAG AGT GAG GTT GCT
CAT CGG TTT AAA GAT TTG GGA GAA GAA AAT TTC AAA GCC TTG
GTG TTG ATT GCC TTT GCT CAG TAT CTT CAG CAG TGT CCA TTT
GAA GAT CAT GTA AAA TTA GTC AAT GAA GTA ACT GAA TTT GCA
AAA ACA TGT GTT GCT GAT GAG TCA GCT GAA AAT TGT GAC AAA
TCA CTT CAT ACC CTT TTT GGA GAC AAA TTA TGC ACA GTT GCA
ACT CTT CGT GAA ACC TAT GGT GAA ATG GCT GAC TGC TGT GCA
AAA CAA GAA CCT GAG AGA AAT GAA TGC TTC TTG CAA CAC AAA
GAT GAC AAC CCA AAC CTC CCC CGA TTG GTG AGA CCA GAG GTT
GAT GTG ATG TGC ACT GCT TTT CAT GAC AAT GAA GAG ACA TTT
TTG AAA AAA TAC TTA TAT GAA ATT GCC AGA AGA CAT CCT TAC
TTT TAT GCC CCG GAA CTC CTT TTC TTT GCT AAA AGG TAT AAA
GCT GCT TTT ACA GAA TGT TGC CAA GCT GCT GAT AAA GCT GCC
TGC CTG TTG CCA AAG CTC GAT GAA CTT CGG GAT GAA GGG AAG
GCT TCG TCT GCC AAA CAG AGA CTC AAG TGT GCC AGT CTC CAA
AAA TTT GGA GAA AGA GCT TTC AAA GCA TGG GCG GTG GCT CGC
CTG AGC CAG AGA TTT CCC AAA GCT GAG TTT GCA GAA GTT TCC
AAG TTA GTG ACA GAT CTT ACC AAA GTC CAC ACG GAA TGC TGC
CAT GGA GAT CTG CTT GAA TGT GCT GAT GAC AGG GCG GAC CTT
GCC AAG TAT ATC TGT GAA AAT CAA GAT TCG ATC TCC AGT AAA
CTG AAG GAA TGC TGT GAA AAA CCT CTG TTG GAA AAA TCC CAC
TGC ATT GCC GAA GTG GAA AAT GAT GAG ATG CCT GCT GAC TTG
CCT TCA TTA GCT GCT GAT TTT GTT GAA AGT AAG GAT GTT TGC
AAA AAC TAT GCT GAG GCA AAG GAT GTC TTC CTG GGC ATG TTT

TTG TAT GAA TAT GCA AGA AGG CAT CCT GAT TAC TCT GTC GTG

CTG CTG CTG AGA CTT GCC AAG ACA TAT GAA ACC ACT CTA GAG

AAG TGC TGT GCC GCT GCA GAT CCT CAT GAA TGC TAT GCC AAA

GTG TTC GAT GAA TTT AAA CCT CCT GTG GAA GAG CCT CAG AAT

TTA ATC AAA CAA AAT TGT GAG CTT TTT GAG CAG CTT GGA GAG

TAC AAA TTC CAG AAT GCG CTA TTA GTT CGT TAC ACC AAG AAA

GTA CCC CAA GTG TCA ACT CCA ACT CTT GTA GAG GTC TCA AGA

AAC CTA GGA AAA GTG GGC AGC AAA TGT TGT AAA CAT CCT GAA

GCA AAA AGA ATG CCC TGT GCA GAA GAC TAT CTA TCC GTG GTC

CTG AAC CAG TTA TGT GTG TTG CAT GAG AAA ACG CCA GTA AGT

GAC AGA GTC ACC AAA TGC TGC ACA GAA TCC TTG GTG AAC AGG

CGA CCA TGC TTT TCA GCT CTG GAA GTC GAT GAA ACA TAC GTT

C CC AAA GAG TTT AAT GCT GAA ACA TTC ACC TTC CAT GCA GAT

ATA TGC ACA CTT TCT GAG AAG GAG AGA CAA ATC AAG AAA CAA

ACT GCA CTT GTT GAG CTC GTG AAA CAC AAG CCC AAG GCA ACA

AAA GAG CAA CTG AAA GCT GTT ATG GAT GAT TTC GCA GCT TTT

GTA GAG AAG TGC TGC AAG GCT GAC GAT AAG GAG ACC TGC TTC

GCC GAG GAG GGT AAA AAA CTT GTT GCT GCA AGT CAA GCT GCC

TTA GGC TTA TAA CATCTACATTTAAAAGCATCTCAGCCTACCATGAGAATA

AGAGAAAGAAAATGAAGATCAAAAGCTTATTCATCTGTTTTCTTTTTCGTTGGTG

TTTTAATCATTTTGCCTCTTTTCTCTGTGCTTCAATTAATAAAAAATGGAAAGAA

TCTAA

wherein the 5' to 3' strand, beginning with the amino terminus is shown, and wherein the abbreviations are defined as in claim 4.

9.  A plasmid having the capability of replication in a prokaryotic or eukaryotic organism, comprising a deoxyribo nucleotide sequence coding for human serum albumin.

10. A plasmid as claimed in claim 8 having the capability of replication in a prokaryotic organism, comprising a human serum albumin or human prepro-serum albumin gene as claimed in any one of claims 1 to 8.

11.   A plasmid as claimed in claim 9 or claim 10
having the capability of replication in a  prokaryotic
organism of the genus Escherichia.

12.   The plasmid of claim 10 designated pGX401
(deposited in E. coli HB101 at the U.S. Dept. of
Agriculture Northern Regional Research Center,
Peoria, Illinois under accession No. NRRL B-15784)
and mutants thereof encoding human serum albumin.

13.   A microorganism transformed by a plasmid as
claimed in any one of claims 9 to 12.

14.   A microorganism as claimed in claim 13 of the
genus Escherichia.

15.   A microorganism as claimed in claim 14 of the
species coli.

16.   A method of producing prepro-human serum albumin
which comprises cultivating on an aqueous nutrient
medium containing assimilable sources of carbon,
nitrogen and essential minerals and growth factors,
under prepro-human serum albumin-producing conditions,
a prokaryotic organism  as claimed in claim 13
transformed by a plasmid capable of replicating
in said organism and having a deoxyribonucleotide sequence
coding for prepro-human serum albumin, and
recovering the prepro-human serum albumin so produced.

17.   A method as claimed in claim 16 wherein the
prokaryotic organism is E. coli.

18.  A method as claimed in claim 17 wherein the prokaryotic organism is transformed by a plasmid substantially similar to plasmid pGX401 as claimed in claim 11.

19.  E. coli strain NRRL No. 15784 (pGX401) or a mutant thereof containing a human prepro-human serum albumin gene.

CLAIMS FOR THE DESIGNATED STATE: AT

1.    A process for preparing a gene coding for human serum albumin (HSA) which comprises obtaining HSA mRNA from HSA-producing cells, in vitro synthesis of complementary DNA (cDNA) using said mRNA as a template and conversion of said cDNA to the double-stranded form.

2.    A process as claimed in claim 1 wherein said gene codes for prepro-human serum albumin.

3.    A process as claimed in claim 1 wherein said gene comprises the following deoxyribonucleotide sequence which corresponds to the indicated amino acid sequence:

```
Asp     Ala     His     Lys     Ser     Glu     Val     Ala
G A Y   G C X   C A Y   A A M   Q R S   G A M   G T X   G C X

His     Arg     Phe     Lys     Asp     Leu     Gly     Glu
C A Y   L G N   T T Y   A A M   G A Y   Y T Z   G G X   G A M

Glu     Asn     Phe     Lys     Ala     Leu     Val     Leu
G A M   A A Y   T T Y   A A M   G C X   Y T Z   G T X   Y T Z

Ile     Ala     Phe     Ala     Gln     Tyr     Leu     Gln
A T H   G C X   T T Y   G C X   C A M   T A Y   Y T Z   C A M

Gln     Cys     Pro     Phe     Glu     Asp     His     Val
C A M   T G Y   C C X   T T Y   G A M   G A Y   C A Y   G T X

Lys     Leu     Val     Asn     Glu     Val     Thr     Glu
A A M   Y T Z   G T X   A A Y   G A M   G T X   A C X   G A M

Phe     Ala     Lys     Thr     Cys     Val     Ala     Asp
T T Y   G C X   A A M   A C X   T G Y   G T X   G C X   G A Y

Glu     Ser     Ala     Glu     Asn     Cys     Asp     Lys
G A M   Q R S   G C X   G A M   A A Y   T G Y   G A Y   A A M

Ser     Leu     His     Thr     Leu     Phe     Gly     Asp
Q R S   Y T Z   C A Y   A C X   Y T Z   T T Y   G G X   G A Y

Lys     Leu     Cys     Thr     Val     Ala     Thr     Leu
A A M   Y T Z   T G Y   A C X   G T X   G C X   A C X   Y T Z

Arg     Glu     Thr     Tyr     Gly     Glu     Met     Ala
L G N   G A M   A C X   T A Y   G G X   G A M   A T G   G C X

Asp     Cys     Cys     Ala     Lys     Gln     Glu     Pro
G A Y   T G Y   T G Y   G C X   A A M   C A M   G A M   C C X
```

0206733

Z

| Glu | Arg | Asn | Glu | Cys | Phe | Leu | Gln |
|-----|-----|-----|-----|-----|-----|-----|-----|
| G A M | L G N | A A Y | G A M | T G Y | T T Y | Y T Z | C A M |

| His | Lys | Asp | Asp | Asn | Pro | Asn | Leu |
|-----|-----|-----|-----|-----|-----|-----|-----|
| C A Y | A A M | G A Y | G A Y | A A Y | C C X | A A Y | Y T Z |

| Pro | Arg | Leu | Val | Arg | Pro | Glu | Val |
|-----|-----|-----|-----|-----|-----|-----|-----|
| C C X | L G N | Y T Z | G T X | L G N | C C X | G A M | G T X |

| Asp | Val | Met | Cys | Thr | Ala | Phe | His |
|-----|-----|-----|-----|-----|-----|-----|-----|
| G A Y | G T X | A T G | T G Y | A C X | G C X | T T Y | C A Y |

| Asp | Asn | Glu | Glu | Thr | Phe | Leu | Lys |
|-----|-----|-----|-----|-----|-----|-----|-----|
| G A Y | A A Y | G A M | G A M | A C X | T T Y | Y T Z | A A M |

| Lys | Tyr | Leu | Tyr | Glu | Ile | Ala | Arg |
|-----|-----|-----|-----|-----|-----|-----|-----|
| A A M | T A Y | Y T Z | T A Y | G A M | A T H | G C X | L G N |

| Arg | His | Pro | Tyr | Phe | Thr | Ala | Pro |
|-----|-----|-----|-----|-----|-----|-----|-----|
| L G N | C A Y | C C X | T A Y | T T Y | A C X | G C X | C C X |

| Glu | Leu | Leu | Phe | Phe | Ala | Lys | Arg |
|-----|-----|-----|-----|-----|-----|-----|-----|
| G A M | Y T Z | Y T Z | T T Y | T T Y | G C X | A A M | L G N |

| Tyr | Lys | Ala | Ala | Phe | Thr | Glu | Cys |
|-----|-----|-----|-----|-----|-----|-----|-----|
| T A Y | A A M | G C X | G C X | T T Y | A C X | G A M | T G Y |

| Cys | Ala | Gln | Ala | Asp | Lys | Ala | Ala |
|-----|-----|-----|-----|-----|-----|-----|-----|
| T G Y | G C X | C A M | G C X | G A Y | A A M | G C X | G C X |

| Cys | Leu | Phe | Pro | Lys | Leu | Asp | Glu |
|-----|-----|-----|-----|-----|-----|-----|-----|
| T G Y | Y T Z | T T Y | C C X | A A M | Y T Z | G A Y | G A M |

| Leu | Arg | Asp | Glu | Gly | Lys | Ala | Ser |
|-----|-----|-----|-----|-----|-----|-----|-----|
| Y T Z | L G N | G A Y | G A M | G G X | A A M | G C X | Q R S |

| Ser | Ala | Lys | Gln | Arg | Leu | Lys | Cys |
|-----|-----|-----|-----|-----|-----|-----|-----|
| Q R S | G C X | A A M | C A M | L G N | Y T Z | A A M | T G Y |

| Ala | Ser | Leu | Gln | Lys | Phe | Gly | Glu |
|-----|-----|-----|-----|-----|-----|-----|-----|
| G C X | Q R S | Y T Z | C A M | A A M | T T Y | G G X | G A M |

| Arg | Ala | Phe | Lys | Ala | Trp | Ala | Val |
|-----|-----|-----|-----|-----|-----|-----|-----|
| L G N | G C X | T T Y | A A M | G C X | T G G | G C X | G T X |

| Ala | Arg | Leu | Ser | Gln | Arg | Phe | Pro |
|-----|-----|-----|-----|-----|-----|-----|-----|
| G C X | L G N | Y T Z | Q R S | C A M | L G N | T T Y | C C X |

| Lys | Ala | Glu | Phe | Ala | Glu | Val | Ser |
|-----|-----|-----|-----|-----|-----|-----|-----|
| A A M | G C X | G A M | T T Y | G C X | G A M | G T X | Q R S |

| Lys | Phe | Val | Thr | Asp | Leu | Thr | Lys |
|-----|-----|-----|-----|-----|-----|-----|-----|
| A A M | T T Y | G T X | A C X | G A Y | Y T Z | A C X | A A M |

| Val | His | Thr | Glu | Cys | Cys | His | Gly |
|-----|-----|-----|-----|-----|-----|-----|-----|
| G T X | C A Y | A C X | G A M | T G Y | T G Y | C A Y | G G X |

3

| Asp | Leu | Leu | Glu | Cys | Ala | Asp | Asp |
|-----|-----|-----|-----|-----|-----|-----|-----|
| G A Y | Y T Z | Y T Z | G A M | T G Y | G C X | G A Y | G A Y |

| Arg | Ala | Asp | Leu | Ala | Lys | Tyr | Ile |
|-----|-----|-----|-----|-----|-----|-----|-----|
| L G N | G C X | G A Y | Y T Z | G C X | A A M | T A Y | A T H |

| Cys | Glu | Asn | Gln | Asp | Ser | Ile | Ser |
|-----|-----|-----|-----|-----|-----|-----|-----|
| T G Y | G A M | A A Y | C A M | G A Y | Q R S | A T H | Q R S |

| Ser | Lys | Leu | Lys | Glu | Cys | Cys | Glu |
|-----|-----|-----|-----|-----|-----|-----|-----|
| Q R S | A A M | Y T Z | A A M | G A M | T G Y | T G Y | G A M |

| Lys | Pro | Leu | Phe | Glu | Lys | Ser | His |
|-----|-----|-----|-----|-----|-----|-----|-----|
| A A M | C C X | Y T Z | T T Y | G A M | A A M | Q R S | C A Y |

| Cys | Ile | Ala | Glu | Val | Glu | Asn | Asp |
|-----|-----|-----|-----|-----|-----|-----|-----|
| T G Y | A T H | G C X | G A M | G T X | G A M | A A Y | G A Y |

| Glu | Met | Pro | Ala | Asp | Phe | Pro | Ser |
|-----|-----|-----|-----|-----|-----|-----|-----|
| G A M | A T G | C C X | G C X | G A Y | T T Y | C C X | Q R S |

| Phe | Ala | Val | Asp | Phe | Val | Glu | Ser |
|-----|-----|-----|-----|-----|-----|-----|-----|
| T T Y | G C X | G T X | G A Y | T T Y | G T X | G A M | Q R S |

| Lys | Asp | Val | Cys | Lys | Asn | Tyr | Ala |
|-----|-----|-----|-----|-----|-----|-----|-----|
| A A M | G A Y | G T X | T G Y | A A M | A A Y | T A Y | G C X |

| Glu | Ala | Lys | Asp | Val | Phe | Leu | Gly |
|-----|-----|-----|-----|-----|-----|-----|-----|
| G A M | G C X | A A M | G A Y | G T X | T T Y | Y T Z | G G X |

| Met | Phe | Phe | Tyr | Glu | Tyr | Ala | Arg |
|-----|-----|-----|-----|-----|-----|-----|-----|
| A T G | T T Y | T T Y | T A Y | G A M | T A Y | G C X | L G N |

| Arg | His | Pro | Asp | Tyr | Ser | Val | Val |
|-----|-----|-----|-----|-----|-----|-----|-----|
| L G N | C A Y | C C X | G A Y | T A Y | Q R S | G T X | G T X |

| Leu | Leu | Leu | Arg | Leu | Ala | Lys | Thr |
|-----|-----|-----|-----|-----|-----|-----|-----|
| Y T Z | Y T Z | Y T Z | L G N | Y T Z | G C X | A A M | A C X |

| Tyr | Glu | Thr | Thr | Leu | Glu | Lys | Cys |
|-----|-----|-----|-----|-----|-----|-----|-----|
| T A Y | G A M | A C X | A C X | Y T Z | G A M | A A M | T G Y |

| Cys | Ala | Ala | Ala | Asp | Pro | His | Glu |
|-----|-----|-----|-----|-----|-----|-----|-----|
| T G Y | G C X | G C X | G C X | G A Y | C C X | C A Y | G A M |

| Cys | Tyr | Ala | Lys | Val | Phe | Asp | Glu |
|-----|-----|-----|-----|-----|-----|-----|-----|
| T G Y | T A Y | G C X | A A M | G T X | T T Y | G A Y | G A M |

| Phe | Lys | Pro | Pro | Val | Glu | Glu | Pro |
|-----|-----|-----|-----|-----|-----|-----|-----|
| T T Y | A A M | C C X | C C X | G T X | G A M | G A M | C C X |

4

| Gln | Asn | Phe | Ile | Lys | Gln | Asn | Cys |
|-----|-----|-----|-----|-----|-----|-----|-----|
| C A M | A A Y | T T Y | A T H | A A M | C A M | A A Y | T G Y |

| Glu | Leu | Phe | Glu | Gln | Leu | Gly | Glu |
|-----|-----|-----|-----|-----|-----|-----|-----|
| G A M | Y T Z | T T Y | G A M | C A M | Y T Z | G G X | G A M |

| Tyr | Lys | Phe | Gln | Asn | Ala | Leu | Phe |
|-----|-----|-----|-----|-----|-----|-----|-----|
| T A Y | A A M | T T Y | C A M | A A Y | G C X | Y T Z | T T Y |

| Val | Arg | Tyr | Thr | Lys | Lys | Val | Pro |
|-----|-----|-----|-----|-----|-----|-----|-----|
| G T X | L G N | T A Y | A C X | A A M | A A M | G T X | C C X |

| Gln | Leu | Ser | Thr | Pro | Thr | Leu | Val |
|-----|-----|-----|-----|-----|-----|-----|-----|
| C A M | Y T Z | Q R S | A C X | C C X | A C X | Y T Z | G T X |

| Glu | Val | Ser | Arg | Asn | Leu | Gly | Lys |
|-----|-----|-----|-----|-----|-----|-----|-----|
| G A M | G T X | Q R S | L G N | A A Y | Y T Z | G G X | A A M |

| Val | Gly | Ser | Lys | Cys | Cys | Lys | His |
|-----|-----|-----|-----|-----|-----|-----|-----|
| G T X | G G X | Q R S | A A M | T G Y | T G Y | A A M | C A Y |

| Pro | Glu | Ala | Lys | Arg | Met | Pro | Cys |
|-----|-----|-----|-----|-----|-----|-----|-----|
| C C X | G A M | G C X | A A M | L G N | A T G | C C X | T G Y |

| Ala | Glu | Asp | Tyr | Leu | Ser | Val | Val |
|-----|-----|-----|-----|-----|-----|-----|-----|
| G C X | G A M | G A Y | T A Y | Y T Z | Q R S | G T X | G T X |

| Leu | Asn | Gln | Leu | Cys | Val | Leu | His |
|-----|-----|-----|-----|-----|-----|-----|-----|
| Y T Z | A A Y | C A M | Y T Z | T G Y | G T X | Y T Z | C A Y |

| Glu | Lys | Thr | Pro | Val | Ser | Asp | Arg |
|-----|-----|-----|-----|-----|-----|-----|-----|
| G A M | A A M | A C X | C C X | G T X | Q R S | G A Y | L G N |

| Val | Thr | Lys | Cys | Cys | Thr | Glu | Ser |
|-----|-----|-----|-----|-----|-----|-----|-----|
| G T X | A C X | A A M | T G Y | T G Y | A C X | G A M | Q R S |

| Leu | Val | Asn | Arg | Arg | Pro | Gly | Phe |
|-----|-----|-----|-----|-----|-----|-----|-----|
| Y T Z | G T X | A A Y | L G N | L G N | C C X | G G X | T T Y |

| Ser | Ala | Leu | Glu | Val | Asp | Glu | Thr |
|-----|-----|-----|-----|-----|-----|-----|-----|
| Q R S | G C X | Y T Z | G A M | G T X | G A Y | G A M | A C X |

| Tyr | Val | Pro | Lys | Glu | Phe | Asn | Ala |
|-----|-----|-----|-----|-----|-----|-----|-----|
| T A Y | G T X | C C X | A A M | G A M | T T Y | A A Y | G C X |

| Glu | Thr | Phe | Thr | Phe | His | Ala | Asp |
|-----|-----|-----|-----|-----|-----|-----|-----|
| G A M | A C X | T T Y | A C X | T T Y | C A Y | G C X | G A Y |

| Ile | Cys | Thr | Leu | Ser | Glu | Lys | Glu |
|-----|-----|-----|-----|-----|-----|-----|-----|
| A T H | T G Y | A C X | Y T Z | Q R S | G A M | A A M | G A M |

```
Arg     Gln     Ile     Lys     Lys     Glu     Thr     Ala
L G N   C A M   A T H   A A M   A A M   G A M   A C X   G C X

Leu     Val     Glu     Leu     Val     Lys     His     Lys
Y T Z   G T X   G A M   Y T Z   G T X   A A M   C A Y   A A M

Pro     Lys     Ala     Thr     Lys     Glu     Glu     Leu
C C X   A A M   G C X   A C X   A A M   G A M   G A M   Y T Z

Lys     Ala     Val     Met     Asp     Asp     Phe     Ala
A A M   G C X   G T X   A T G   G A Y   G A Y   T T Y   G C X

Ala     Phe     Val     Glu     Lys     Cys     Cys     Lys
G C X   T T Y   G T X   G A M   A A M   T G Y   T G Y   A A M

Ala     Asp     Asp     Lys     Glu     Thr     Cys     Phe
G C X   G A Y   G A Y   A A M   G A M   A C X   T G Y   T T Y

Ala     Glu     Glu     Gly     Lys     Lys     Leu     Val
G C X   G A M   G A M   G G X   A A M   A A M   Y T Z   G T X

Ala     Ala     Ser     Glu     Ala     Val     Leu     Gly
G C X   G C X   Q R S   G A M   G C X   G T X   Y T Z   G G X

Leu
Y T Z   T A A
```

wherein, the 5' to 3' strand, beginning with the amino terminus and the amino acids for which each triplet codes are shown, and wherein the abbreviations have the following standard meanings:

A is deoxyadenyl

T is thymidyl

G is deoxyguanyl

C is deoxycytosyl

X is A, T, C or G

Y is T or C

When Y is C, Z is A, T, C or G

When Y is T, Z is A or G

H is A, T or C

Q is T or A

When Q is T, R is C and S is A, T, C or G

When Q is A, R is G and S is T or C

```
M is A or G
L is A or C
When L is A, N is A or G
When L is C, N is A, T, C or G
GLY is glycine
ALA is alanine
VAL is valine
LEU is leucine
ILE is isoleucine
SER is serine
THR is threonine
PHE is phenylalanine
TYR is tyrosine
TRP is tyryptophan
CYS is cysteine
MET is methionine
ASP is aspartic acid
GLU is glutamic acid
LYS is lysine
ARG is arginine
HIS is histidine
PRO is proline
GLN is glutamine
ASN is asparagine
```

4.     A process as claimed in claim 2 wherein said gene comprises the following deoxyribonucleotide sequence:

```
     Met     Lys     Trp     Val     Thr     Phe
     A T G   A A M   T G G   G T X   A C X   T T Y

Ile     Ser     Leu     Leu     Phe     Leu     Phe
A T H   Q R S   Y T Z   Y T Z   T T Y   Y T Z   T T Y

Ser     Ser     Ala     Tyr     Ser     Arg     Gly
Q R S   Q R S   G C X   T A Y   Q R S   L G N   G G X

Val     Phe     Arg     Arg     Asp     Ala     His     Lys
G T X   T T Y   L G N   L G N   G A Y   G C X   C A Y   A A M
```

0206733

```
Ser     Glu     Val     Ala     His     Arg     Phe     Lys
Q R S   G A M   G T X   G C X   C A Y   L G N   T T Y   A A M

Asp     Leu     Gly     Glu     Glu     Asn     Phe     Lys
G A Y   Y T Z   G G X   G A M   G A M   A A Y   T T Y   A A M

Ala     Leu     Val     Leu     Ile     Ala     Phe     Ala
G C X   Y T Z   G T X   Y T Z   A T H   G C X   T T Y   G C X

Gln     Tyr     Leu     Gln     Gln     Cys     Pro     Phe
C A M   T A Y   Y T Z   C A M   C A M   T G Y   C C X   T T Y

Glu     Asp     His     Val     Lys     Leu     Val     Asn
G A M   G A Y   C A Y   G T X   A A M   Y T Z   G T X   A A Y

Glu     Val     Thr     Glu     Phe     Ala     Lys     Thr
G A M   G T X   A C X   G A M   T T Y   G C X   A A M   A C X

Cys     Val     Ala     Asp     Glu     Ser     Ala     Glu
T G Y   G T X   G C X   G A Y   G A M   Q R S   G C X   G A M

Asn     Cys     Asp     Lys     Ser     Leu     His     Thr
A A Y   T G Y   G A Y   A A M   Q R S   Y T Z   C A Y   A C X

Leu     Phe     Gly     Asp     Lys     Leu     Cys     Thr
Y T Z   T T Y   G G X   G A Y   A A M   Y T Z   T G Y   A C X

Val     Ala     Thr     Leu     Arg     Glu     Thr     Tyr
G T X   G C X   A C X   Y T Z   L G N   G A M   A C X   T A Y

Gly     Glu     Met     Ala     Asp     Cys     Cys     Ala
G G X   G A M   A T G   G C X   G A Y   T G Y   T G Y   G C X

Lys     Gln     Glu     Pro     Glu     Arg     Asn     Glu
A A M   C A M   G A M   C C X   G A M   L G N   A A Y   G A M

Cys     Phe     Leu     Gln     His     Lys     Asp     Asp
T G Y   T T Y   Y T Z   C A M   C A Y   A A M   G A Y   G A Y

Asn     Pro     Asn     Leu     Pro     Arg     Leu     Val
A A Y   C C X   A A Y   Y T Z   C C X   L G N   Y T Z   G T X

Arg     Pro     Glu     Val     Asp     Val     Met     Cys
L G N   C C X   G A M   G T X   G A Y   G T X   A T G   T G Y

Thr     Ala     Phe     His     Asp     Asn     Glu     Glu
A C X   G C X   T T Y   C A Y   G A Y   A A Y   G A M   G A M

Thr     Phe     Leu     Lys     Lys     Tyr     Leu     Tyr
A C X   T T Y   Y T Z   A A M   A A M   T A Y   Y T Z   T A Y
```

```
Glu      Ile      Ala      Arg      Arg      His      Pro      Tyr
G A M    A T H    G C X    L G N    L G N    C A Y    C C X    T A Y

Phe      Thr      Ala      Pro      Glu      Leu      Leu      Phe
T T Y    A C X    G C X    C C X    G A M    Y T Z    Y T Z    T T Y

Phe      Ala      Lys      Arg      Tyr      Lys      Ala      Ala
T T Y    G C X    A A M    L G N    T A Y    A A M    G C X    G C X

Phe      Thr      Glu      Cys      Cys      Ala      Gln      Ala
T T Y    A C X    G A M    T G Y    T G Y    G C X    C A M    G C X

Asp      Lys      Ala      Ala      Cys      Leu      Phe      Pro
G A Y    A A M    G C X    G C X    T G Y    Y T Z    T T Y    C C X

Lys      Leu      Asp      Glu      Leu      Arg      Asp      Glu
A A M    Y T Z    G A Y    G A M    Y T Z    L G N    G A Y    G A M

Gly      Lys      Ala      Ser      Ser      Ala      Lys      Gln
G G X    A A M    G C X    Q R S    Q R S    G C X    A A M    C A M

Arg      Leu      Lys      Cys      Ala      Ser      Leu      Gln
L G N    Y T Z    A A M    T G Y    G C X    Q R S    Y T Z    C A M

Lys      Phe      Gly      Glu      Arg      Ala      Phe      Lys
A A M    T T Y    G G X    G A M    L G N    G C X    T T Y    A A M

Ala      Trp      Ala      Val      Ala      Arg      Leu      Ser
G C X    T G G    G C X    G T X    G C X    L G N    Y T Z    Q R S

Gln      Arg      Phe      Pro      Lys      Ala      Glu      Phe
C A M    L G N    T T Y    C C X    A A M    G C X    G A M    T T Y

Ala      Glu      Val      Ser      Lys      Phe      Val      Thr
G C X    G A M    G T X    Q R S    A A M    T T Y    G T X    A C X

Asp      Leu      Thr      Lys      Val      His      Thr      Glu
G A Y    Y T Z    A C X    A A M    G T X    C A Y    A C X    G A M

Cys      Cys      His      Gly      Asp      Leu      Leu      Glu
T G Y    T G Y    C A Y    G G X    G A Y    Y T Z    Y T Z    G A M

Cys      Ala      Asp      Asp      Arg      Ala      Asp      Leu
T G Y    G C X    G A Y    G A Y    L G N    G C X    G A Y    Y T Z

Ala      Lys      Tyr      Ile      Cys      Glu      Asn      Gln
G C X    A A M    T A Y    A T H    T G Y    G A M    A A Y    C A M

Asp      Ser      Ile      Ser      Ser      Lys      Leu      Lys
G A Y    Q R S    A T H    Q R S    Q R S    A A M    Y T Z    A A M
```

```
Glu     Cys     Cys     Glu     Lys     Pro     Leu     Phe
G A M   T G Y   T G Y   G A M   A A M   C C X   Y T Z   T T Y

Glu     Lys     Ser     His     Cys     Ile     Ala     Glu
G A M   A A M   Q R S   C A Y   T G Y   A T H   G C X   G A M

Val     Glu     Asn     Asp     Glu     Met     Pro     Ala
G T X   G A M   A A Y   G A Y   G A M   A T G   C C X   G C X

Asp     Phe     Pro     Ser     Phe     Ala     Val     Asp
G A Y   T T Y   C C X   Q R S   T T Y   G C X   G T X   G A Y

Phe     Val     Glu     Ser     Lys     Asp     Val     Cys
T T Y   G T X   G A M   Q R S   A A M   G A Y   G T X   T G Y

Lys     Asn     Tyr     Ala     Glu     Ala     Lys     Asp
A A M   A A Y   T A Y   G C X   G A M   G C X   A A M   G A Y

Val     Phe     Leu     Gly     Met     Phe     Phe     Tyr
G T X   T T Y   Y T Z   G G X   A T G   T T Y   T T Y   T A Y

Glu     Tyr     Ala     Arg     Arg     His     Pro     Asp
G A M   T A Y   G C X   L G N   L G N   C A Y   C C X   G A Y

Tyr     Ser     Val     Val     Leu     Leu     Leu     Arg
T A Y   Q R S   G T X   G T X   Y T Z   Y T Z   Y T Z   L G N

Leu     Ala     Lys     Thr     Tyr     Glu     Thr     Thr
Y T Z   G C X   A A M   A C X   T A Y   G A M   A C X   A C X

Leu     Glu     Lys     Cys     Cys     Ala     Ala     Ala
Y T Z   G A M   A A M   T G Y   T G Y   G C X   G C X   G C X

Asp     Pro     His     Glu     Cys     Tyr     Ala     Lys
G A Y   C C X   C A Y   G A M   T G Y   T A Y   G C X   A A M

Val     Phe     Asp     Glu     Phe     Lys     Pro     Pro
G T X   T T Y   G A Y   G A M   T T Y   A A M   C C X   C C X

Val     Glu     Glu     Pro     Gln     Asn     Phe     Ile
G T X   G A M   G A M   C C X   C A M   A A Y   T T Y   A T H

Lys     Gln     Asn     Cys     Glu     Leu     Phe     Glu
A A M   C A M   A A Y   T G Y   G A M   Y T Z   T T Y   G A M

Gln     Leu     Gly     Glu     Tyr     Lys     Phe     Gln
C A M   Y T Z   G G X   G A M   T A Y   A A M   T T Y   C A M

Asn     Ala     Leu     Phe     Val     Arg     Tyr     Thr
A A Y   G C X   Y T Z   T T Y   G T X   L G N   T A Y   A C X
```

```
Lys     Lys     Val     Pro     Gln     Leu     Ser     Thr
A A M   A A M   G T X   C C X   C A M   Y T Z   Q R S   A C X

Pro     Thr     Leu     Val     Glu     Val     Ser     Arg
C C X   A C X   Y T Z   G T X   G A M   G T X   Q R S   L G N

Asn     Leu     Gly     Lys     Val     Gly     Ser     Lys
A A Y   Y T Z   G G X   A A M   G T X   G G X   Q R S   A A M

Cys     Cys     Lys     His     Pro     Glu     Ala     Lys
T G Y   T G Y   A A M   C A Y   C C X   G A M   G C X   A A M

Arg     Met     Pro     Cys     Ala     Glu     Asp     Tyr
L G N   A T G   C C X   T G Y   G C X   G A M   G A Y   T A Y

Leu     Ser     Val     Val     Leu     Asn     Gln     Leu
Y T Z   Q R S   G T X   G T X   Y T Z   A A Y   C A M   Y T Z

Cys     Val     Leu     His     Glu     Lys     Thr     Pro
T G Y   G T X   Y T Z   C A Y   G A M   A A M   A C X   C C X

Val     Ser     Asp     Arg     Val     Thr     Lys     Cys
G T X   Q R S   G A Y   L G N   G T X   A C X   A A M   T G Y

Cys     Thr     Glu     Ser     Leu     Val     Asn     Arg
T G Y   A C X   G A M   Q R S   Y T Z   G T X   A A Y   L G N

Arg     Pro     Gly     Phe     Ser     Ala     Leu     Glu
L G N   C C X   G G X   T T Y   Q R S   G C X   Y T Z   G A M

Val     Asp     Glu     Thr     Tyr     Val     Pro     Lys
G T X   G A Y   G A M   A C X   T A Y   G T X   C C X   A A M

Glu     Phe     Asn     Ala     Glu     Thr     Phe     Thr
G A M   T T Y   A A Y   G C X   G A M   A C X   T T Y   A C X

Phe     His     Ala     Asp     Ile     Cys     Thr     Leu
T T Y   C A Y   G C X   G A Y   A T H   T G Y   A C X   Y T Z

Ser     Glu     Lys     Glu     Arg     Gln     Ile     Lys
Q R S   G A M   A A M   G A M   L G N   C A M   A T H   A A M

Lys     Glu     Thr     Ala     Leu     Val     Glu     Leu
A A M   G A M   A C X   G C X   Y T Z   G T X   G A M   Y T Z

Val     Lys     His     Lys     Pro     Lys     Ala     Thr
G T X   A A M   C A Y   A A M   C C X   A A M   G C X   A C X

Lys     Glu     Glu     Leu     Lys     Ala     Val     Met
A A M   G A M   G A M   Y T Z   A A M   G C X   G T X   A T G

Asp     Asp     Phe     Ala     Ala     Phe     Val     Glu
G A Y   G A Y   T T Y   G C X   G C X   T T Y   G T X   G A M
```

II

| Lys | Cys | Cys | Lys | Ala | Asp | Asp | Lys |
|-----|-----|-----|-----|-----|-----|-----|-----|
| A A M | T G Y | T G Y | A A M | G C X | G A Y | G A Y | A A M |

| Glu | Thr | Cys | Phe | Ala | Glu | Glu | Gly |
|-----|-----|-----|-----|-----|-----|-----|-----|
| G A M | A C X | T G Y | T T Y | G C X | G A M | G A M | G G X |

| Lys | Lys | Leu | Val | Ala | Ala | Ser | Glu |
|-----|-----|-----|-----|-----|-----|-----|-----|
| A A M | A A M | Y T Z | G T X | G C X | G C X | Q R S | G A M |

| Ala | Val | Leu | Gly | Leu | |
|-----|-----|-----|-----|-----|-----|
| G C X | G T X | Y T Z | G G X | Y T Z | T A A |

wherein the 5' and 3' strand, beginning with the amino terminus, and the amino acids for which each triplet codes are shown, and wherein the abbreviations are defined as in claim 3.

5.    A process as claimed in claim 3 wherein said gene comprises the following deoxyribonucleotide sequence:

GAT GCA CAC AAG AGT GAG GTT GCT CAT CGG TTT AAA GAT TTG

GGA GAA GAA AAT TTC AAA GCC TTG GTG TTG ATT GCC TTT GCT

CAG TAT CTT CAG CAG TGT CCA TTT GAA GAT CAT GTA AAA TTA

GTG AAT GAA GTA ACT GAA TTT GCA AAA ACA TGT GTT GCT GAT

GAG TCA GCT GAA AAT TGT GAC AAA TCA CTT CAT ACC CTT TTT

GGA GAC AAA TTA TGC ACA GTT GCA ACT CTT CGT GAA ACC TAT

GGT GAA ATG GCT GAC TGC TGT GCA AAA CAA GAA CCT GAG AGA

AAT GAA TGC TTC TTG CAA CAC AAA GAT GAC AAC CCA AAC CTC

CCC CGA TTG GTG AGA CCA GAG GTT GAT GTG ATG TGC ACT GCT

TTT CAT GAC AAT GAA GAG ACA TTT TTG AAA AAA TAC TTA TAT

GAA ATT GCC AGA AGA CAT CCT TAC TTT TAT GCC CCG GAA CTC

CTT TTC TTT GCT AAA AGG TAT AAA GCT GCT TTT ACA GAA TGT

TGC CAA GCT GCT GAT AAA GCT GCC TGC CTG TTG CCA AAG CTC

GAT GAA CTT CGG GAT GAA GGG AAG GCT TCG TCT GCC AAA CAG

AGA CTC AAG TGT GCC AGT CTC CAA AAA TTT GGA GAA AGA GCT

TTC AAA GCA TGG GCG GTG GCT CGC CTG AGC CAG AGA TTT CCC

AAA GCT GAG TTT GCA GAA GTT TCC AAG TTA GTG ACA GAT CTT

ACC AAA GTC CAC ACG GAA TGC TGC CAT GGA GAT CTG CTT GAA

TGT GCT GAT GAC AGG GCG GAC CTT GCC AAG TAT ATC TGT GAA

AAT CAA GAT TCG ATC TCC AGT AAA CTG AAG GAA TGC TGT GAA

```
AAA CCT CTG TTG GAA AAA TCC CAC TGC ATT GCC GAA GTG GAA
AAT GAT GAG ATG CCT GCT GAC TTG CCT TCA TTA GCT GCT GAT
TTT GTT GAA AGT AAG GAT GTT TGC AAA AAC TAT GCT GAG GCA
AAG GAT GTC TTC CTG GGC ATG TTT TTG TAT GAA TAT GCA AGA
AGG CAT CCT GAT TAC TCT GTC GTG CTG CTG CTG AGA CTT GCC
AAG ACA TAT GAA ACC ACT CTA GAG AAG TGC TGT GCC GCT GCA
GAT CCT CAT GAA TGC TAT GCC AAA GTG TTC GAT GAA TTT AAA
CCT CCT GTG GAA GAG CCT CAG AAT TTA ATC AAA CAA AAT TGT
GAG CTT TTT GAG CAG CTT GGA GAG TAC AAA TTC CAG AAT GCG
CTA TTA GTT CGT TAC ACC AAG AAA GTA CCC CAA GTG TCA ACT
CCA ACT CTT GTA GAG GTC TCA AGA AAC CTA GGA AAA GTG GGC
AGC AAA TGT TGT AAA CAT CCT GAA GCA AAA AGA ATG CCC TGT
GCA GAA GAC TAT CTA TCC GTG GTC CTG AAC CAG TTA TGT GTG
TTG CAT GAG AAA ACG CCA GTA AGT GAC AGA GTC ACC AAA TGC
TGC ACA GAA TCC TTG GTG AAC AGG CGA CCA TGC TTT TCA GCT
CTG GAA GTC GAT GAA ACA TAC GTT CCC AAA GAG TTT AAT GCT
GAA ACA TTC ACC TTC CAT GCA GAT ATA TGC ACA CTT TCT GAG
AAG GAG AGA CAA ATC AAG AAA CAA ACT GCA CTT GTT GAG CTC
GTG AAA CAC AAG CCC AAG GCA ACA AAA GAG CAA CTG AAA GCT
GTT ATG GAT GAT TTC GCA GCT TTT GTA GAG AAG TGC TGC AAG
GCT GAC GAT AAG GAG ACC TGC TTT GCC GAG GAG GGT AAA AAA
CTT GTT GCT GCA AGT CAA GCT GCC TTA GGC TTA TAA
```
wherein the 5' to 3' strand, beginning with the amino
terminus is shown, and wherein the abbreviations are
defined as in claim 3.

6.    A process as claimed in claim 4 wherein said
gene comprises the following deoxyribonucleotide
sequence:
```
ATG AAG TGG GTA ACC TTT ATT TCC CTT CTT TTT CTC TTT AGC
TCG GCT TAT TCC AGG GGT GTG TTT CGT CGA GAT GCA CAC AAG
AGT GAG GTT GCT CAT CGG TTT AAA GAT TTG GGA GAA GAA AAT
TTC AAA GCC TTG GTG TTG ATT GCC TTT GCT CAG TAT CTT CAG
CAG TGT CCA TTT GAA GAT CAT GTA AAA TTA GTG AAT GAA GTA
ACT GAA TTT GCA AAA ACA TGT GTT GCT GAT GAG TCA GCT GAA
```

```
AAT TGT GAC AAA TCA CTT CAT ACC CTT TTT GGA GAC AAA TTA
TGC ACA GTT GCA ACT CTT CGT GAA ACC TAT GGT GAA ATG GCT
GAC TGC TGT GCA AAA CAA GAA CCT GAG AGA AAT GAA TGC TTC
TTG CAA CAC AAA GAT GAC AAC CCA AAC CTC CCC CGA TTG GTG
AGA CCA GAG GTT GAT GTG ATG TGC ACT GCT TTT CAT GAC AAT
GAA GAG ACA TTT TTG AAA AAA TAC TTA TAT GAA ATT GCC AGA
AGA CAT CCT TAC TTT TAT GCC CCG GAA CTC CTT TTC TTT GCT
AAA AGG TAT AAA GCT GCT TTT ACA GAA TGT TGC CAA GCT GCT
GAT AAA GCT GCC TGC CTG TTG CCA AAG CTC GAT GAA CTT CGG
GAT GAA GGG AAG GCT TCG TCT GCC AAA CAG AGA CTC AAG TGT
GCC AGT CTC CAA AAA TTT GGA GAA AGA GCT TTC AAA GCA TGG
GCG GTG GCT CGC CTG AGC CAG AGA TTT CCC AAA GCT GAG TTT
GCA GAA GTT TCC AAG TTA GTG ACA GAT CTT ACC AAA GTC CAC
ACG GAA TGC TGC CAT GGA GAT CTG CTT GAA TGT GCT GAT GAC
AGG GCG GAC CTT GCC AAG TAT ATC TGT GAA AAT CAA GAT TCG
ATC TCC AGT AAA CTG AAG GAA TGC TGT GAA AAA CCT CTG TTG
GAA AAA TCC CAC TGC ATT GCC GAA GTG GAA AAT GAT GAG ATG
CCT GCT GAC TTG CCT TCA TTA GCT GCT GAT TTT GTT GAA AGT
AAG GAT GTT TGC AAA AAC TAT GCT GAG GCA AAG GAT GTC TTC
CTG GGC ATG TTT TTG TAT GAA TAT GCA AGA AGG CAT CCT GAT
TAC TCT GTC GTG CTG CTG CTG AGA CTT GCC AAG ACA TAT GAA
ACC ACT CTA GAG AAG TGC TGT GCC GCT GCA GAT CCT CAT GAA
TGC TAT GCC AAA GTG TTC GAT GAA TTT AAA CCT CCT GTG GAA
GAG CCT CAG AAT TTA ATC AAA CAA AAT TGT GAG CTT TTT GAG
CAG CTT GGA GAG TAC AAA TTC CAG AAT GCG CTA TTA GTT CGT
TAC ACC AAG AAA GTA CCC CAA GTG TCA ACT CCA ACT CTT GTA
GAG GTC TCA AGA AAC CTA GGA AAA GTG GGC AGC AAA TGT TGT
AAA CAT CCT GAA GCA AAA AGA ATG CCC TGT GCA GAA GAC TAT
CTA TCC GTG GTC CTG AAC CAG TTA TGT GTG TTG CAT GAG AAA
ACG CCA GTA AGT GAC AGA GTC ACC AAA TGC TGC ACA GAA TCC
TTG GTG AAC AGG CGA CCA TGC TTT TCA GCT CTG GAA GTC GAT
GAA ACA TAC GTT CCC AAA GAG TTT AAT GCT GAA ACA TTC ACC
TTC CAT GCA GAT ATA TGC ACA CTT TCT GAG AAG GAG AGA CAA
ATC AAG AAA CAA ACT GCA CTT GTT GAG CTC GTG AAA CAC AAG
CCC AAG GCA ACA AAA GAG CAA CTG AAA GCT GTT ATG GAT GAT
```

TTC GCA GCT TTT GTA GAG AAG TGC TGC AAG GCT GAC GAT AAG
GAG ACC TGC TTT GCC GAG GAG GGT AAA AAA CTT GTT GCT GCA
AGT CAA GCT GCC TTA GGC TTA TAA
wherein the 5' to 3' strand, beginning with the amino
terminus is shown, and wherein the abbreviations are
defined as in claim 3.

7.    A process as claimed in claim 6 wherein said
gene is comprised in the following deoxyribonucleotide
sequence:
5'

   TCTCTTCTGTCAACCCCACGCCTTTGGCACA ATG AAG TGG GTA
ACC TTT ATT TCC CTT CTT TTT CTC TTT AGC TCG GCT TAT TCC
AGG GGT GTG TTT CGT CGA GAT GCA CAC AAG AGT GAG GTT GCT
CAT CGG TTT AAA GAT TTG GGA GAA GAA AAT TTC AAA GCC TTG
GTG TTG ATT GCC TTT GCT CAG TAT CTT CAG CAG TGT CCA TTT
GAA GAT CAT GTA AAA TTA GTC AAT GAA GTA ACT GAA TTT GCA
AAA ACA TGT GTT GCT GAT GAG TCA GCT GAA AAT TGT GAC AAA
TCA CTT CAT ACC CTT TTT GGA GAC AAA TTA TGC ACA GTT GCA
ACT CTT CGT GAA ACC TAT GGT GAA ATG GCT GAC TGC TGT GCA
AAA CAA GAA CCT GAG AGA AAT GAA TGC TTC TTG CAA CAC AAA
GAT GAC AAC CCA AAC CTC CCC CGA TTG GTG AGA CCA GAG GTT
GAT GTG ATG TGC ACT GCT TTT CAT GAC AAT GAA GAG ACA TTT
TTG AAA AAA TAC TTA TAT GAA ATT GCC AGA AGA CAT CCT TAC
TTT TAT GCC CCG GAA CTC CTT TTC TTT GCT AAA AGG TAT AAA
GCT GCT TTT ACA GAA TGT TGC CAA GCT GCT GAT AAA GCT GCC
TGC CTG TTG CCA AAG CTC GAT GAA CTT CGG GAT GAA GGG AAG
GCT TCG TCT GCC AAA CAG AGA CTC AAG TGT GCC AGT CTC CAA
AAA TTT GGA GAA AGA GCT TTC AAA GCA TGG GCG GTG GCT CGC
CTG AGC CAG AGA TTT CCC AAA GCT GAG TTT GCA GAA GTT TCC
AAG TTA GTG ACA GAT CTT ACC AAA GTC CAC ACG GAA TGC TGC
CAT GGA GAT CTG CTT GAA TGT GCT GAT GAC AGG GCG GAC CTT
GCC AAG TAT ATC TGT GAA AAT CAA GAT TCG ATC TCC AGT AAA
CTG AAG GAA TGC TGT GAA AAA CCT CTG TTG GAA AAA TCC CAC
TGC ATT GCC GAA GTG GAA AAT GAT GAG ATG CCT GCT GAC TTG
CCT TCA TTA GCT GCT GAT TTT GTT GAA AGT AAG GAT GTT TGC
AAA AAC TAT GCT GAG GCA AAG GAT GTC TTC CTG GGC ATG TTT

TTG TAT GAA TAT GCA AGA AGG CAT CCT GAT TAC TCT GTC GTG
CTG CTG CTG AGA CTT GCC AAG ACA TAT GAA ACC ACT CTA GAG
AAG TGC TGT GCC GCT GCA GAT CCT CAT GAA TGC TAT GCC AAA
GTG TTC GAT GAA TTT AAA CCT CCT GTG GAA GAG CCT CAG AAT
TTA ATC AAA CAA AAT TGT GAG CTT TTT GAG CAG CTT GGA GAG
TAC AAA TTC CAG AAT GCG CTA TTA GTT CGT TAC ACC AAG AAA
GTA CCC CAA GTG TCA ACT CCA ACT CTT GTA GAG GTC TCA AGA
AAC CTA GGA AAA GTG GGC AGC AAA TGT TGT AAA CAT CCT GAA
GCA AAA AGA ATG CCC TGT GCA GAA GAC TAT CTA TCC GTG GTC
CTG AAC CAG TTA TGT GTG TTG CAT GAG AAA ACG CCA GTA AGT
GAC AGA GTC ACC AAA TGC TGC ACA GAA TCC TTG GTG AAC AGG
CGA CCA TGC TTT TCA GCT CTG GAA GTC GAT GAA ACA TAC GTT
CCC AAA GAG TTT AAT GCT GAA ACA TTC ACC TTC CAT GCA GAT
ATA TGC ACA CTT TCT GAG AAG GAG AGA CAA ATC AAG AAA CAA
ACT GCA CTT GTT GAG CTC GTG AAA CAC AAG CCC AAG GCA ACA
AAA GAG CAA CTG AAA GCT GTT ATG GAT GAT TTC GCA GCT TTT
GTA GAG AAG TGC TGC AAG GCT GAC GAT AAG GAG ACC TGC TTC
GCC GAG GAG GGT AAA AAA CTT GTT GCT GCA AGT CAA GCT GCC
TTA GGC TTA TAA CATCTACATTTAAAAGCATCTCAGCCTACCATGAGAATA
AGAGAAAGAAAATGAAGATCAAAAGCTTATTCATCTGTTTTCTTTTTCGTTGGTG
TTTTAATCATTTTGCCTCTTTTCTCTGTGCTTCAATTAATAAAAAATGGAAAGAA
TCTAA

wherein the 5' to 3' strand, beginning with the amino
terminus is shown, and wherein the abbreviations are
defined as in claim 3.

8.    A process for preparing a plasmid encoding human
serum albumin which comprises inserting a deoxyribonucleotide sequence coding for human serum albumin
into a plasmid  having the capability of replication
in a prokaryotic or eukaryotic organism.


9.    A process as claimed in claim 8 wherein the
deoxyribonucleotide sequence coding for human serum
albumin is prepared by a process as claimed in
any one of claims 1 to 7.

16

10. A process as claimed in claim 8 or claim 9 wherein the deoxyribonucleotide sequence coding for human serum alubmin is inserted into a plasmid having the capability of replication in a prokaryotic organism of the genus Escherichia.

11. The process of claim 10 wherein the deoxyribonucleotide sequence of claim 8 is inserted at the Pst I site of plasmid pBR322 so as to prepare plasmid pGX401.

12. A process for preparing a microorganism containing a gene coding for human serum albumin which comprises transforming a microorganism with a plasmid capable of replicating in said microorganism and including said gene.

13. A process as claimed in claim 12 wherein said plasmid is prepared by a process as claimed in any one of claims 8 to 11.

14. A microorganism transformed by a plasmid containing a deoxyribonucleotide sequence as defined in any one of claims 3 to 7.

15. A microorganism as claimed in claim 14 of the genus Escherichia.

16. A method of producing prepro-human serum albumin which comprises cultivating on an aqueous nutrient medium containing assimilable sources of carbon, nitrogen and essential minerals and growth factors, under prepro-human serum albumin-producing conditions, a prokaryotic organism transformed by a plasmid capable of replicating in said organism and having a deoxyribonucleotide sequence coding for

17

prepro-human serum albumin, and recovering the prepro-human serum albumin so produced.

17. A method as claimed in claim 16 wherein the prokaryotic organism is E. coli.

18. A method as claim in claim 17 wherein the prokaryotic organism is transformed by a plasmid substantially similar to plasmid pGX401.

19. E. coli strain NRRL No. 15784 (pGX401), or a mutant thereof containing a human prepro-human serum albumin gene.

FIG. 1.

PARTIAL RESTRICTION MAPS OF HSA cDNA IN pGX401

FIG. 4

(5' Pst I SITE WAS NOT REGENERATED)

Figure 2

Complete Nucleotide Sequence of the
HSA Insert In Clone pGX401

```
                                         |<——————————————
5'                              Met Lys Trp Val
   TCTCTTCTGTCAACCCCACGCCTTTGGCACA ATG AAG TGG GTA

——————————————————————— pre HSA ———————————————————>|
Thr Phe Ile Ser Leu Leu Phe Leu Phe Ser Ser Ala Tyr Ser
ACC TTT ATT TCC CTT CTT TTT CTC TTT AGC TCG GCT TAT TCC

|<——— pro HSA ———>|
Arg Gly Val Phe Arg Arg Asp Ala His Lys Ser Glu Val Ala
AGG GGT GTG TTT CGT CGA GAT GCA CAC AAG AGT GAG GTT GCT

His Arg Phe Lys Asp Leu Gly Glu Glu Asn Phe Lys Ala Leu
CAT CGG TTT AAA GAT TTG GGA GAA GAA AAT TTC AAA GCC TTG

Val Leu Ile Ala Phe Ala Gln Tyr Leu Gln Gln Cys Pro Phe
GTG TTG ATT GCC TTT GCT CAG TAT CTT CAG CAG TGT CCA TTT

Glu Asp His Val Lys Leu Val Asn Glu Val Thr Glu Phe Ala
GAA GAT CAT GTA AAA TTA GTC AAT GAA GTA ACT GAA TTT GCA

Lys Thr Cys Val Ala Asp Glu Ser Ala Glu Asn Cys Asp Lys
AAA ACA TGT GTT GCT GAT GAG TCA GCT GAA AAT TGT GAC AAA

Ser Leu His Thr Leu Phe Gly Asp Lys Leu Cys Thr Val Ala
TCA CTT CAT ACC CTT TTT GGA GAC AAA TTA TGC ACA GTT GCA

Thr Leu Arg Glu Thr Tyr Gly Glu Met Ala Asp Cys Cys Ala
ACT CTT CGT GAA ACC TAT GGT GAA ATG GCT GAC TGC TGT GCA

Lys Gln Glu Pro Glu Arg Asn Glu Cys Phe Leu Gln His Lys
AAA CAA GAA CCT GAG AGA AAT GAA TGC TTC TTG CAA CAC AAA

Asp Asp Asn Pro Asn Leu Pro Arg Leu Val Arg Pro Glu Val
GAT GAC AAC CCA AAC CTC CCC CGA TTG GTG AGA CCA GAG GTT

Asp Val Met Cys Thr Ala Phe His Asp Asn Glu Glu Thr Phe
GAT GTG ATG TGC ACT GCT TTT CAT GAC AAT GAA GAG ACA TTT

Leu Lys Lys Tyr Leu Tyr Glu Ile Ala Arg Arg His Pro Tyr
TTG AAA AAA TAC TTA TAT GAA ATT GCC AGA AGA CAT CCT TAC

Phe Thr Ala Pro Glu Leu Leu Phe Phe Ala Lys Arg Tyr Lys
TTT TAT GCC CCG GAA CTC CTT TTC TTT GCT AAA AGG TAT AAA
```

Figure 2 (continued)

```
Ala Ala Phe Thr Glu Cys Cys Ala Gln Ala Asp Lys Ala Ala
GCT GCT TTT ACA GAA TGT TGC CAA GCT GCT GAT AAA GCT GCC

Cys Leu Phe Pro Lys Leu Asp Glu Leu Arg Asp Glu Gly Lys
TGC CTG TTG CCA AAG CTC GAT GAA CTT CGG GAT GAA GGG AAG

Ala Ser Ser Ala Lys Gln Arg Leu Lys Cys Ala Ser Leu Gln
GCT TCG TCT GCC AAA CAG AGA CTC AAG TGT GCC AGT CTC CAA

Lys Phe Gly Glu Arg Ala Phe Lys Ala Trp Ala Val Ala Arg
AAA TTT GGA GAA AGA GCT TTC AAA GCA TGG GCG GTG GCT CGC

Leu Ser Gln Arg Phe Pro Lys Ala Glu Phe Ala Glu Val Ser
CTG AGC CAG AGA TTT CCC AAA GCT GAG TTT GCA GAA GTT TCC

Lys Phe Val Thr Asp Leu Thr Lys Val His Thr Glu Cys Cys
AAG TTA GTG ACA GAT CTT ACC AAA GTC CAC ACG GAA TGC TGC

His Gly Asp Leu Leu Glu Cys Ala Asp Asp Arg Ala Asp Leu
CAT GGA GAT CTG CTT GAA TGT GCT GAT GAC AGG GCG GAC CTT

Ala Lys Tyr Ile Cys Glu Asn Gln Asp Ser Ile Ser Ser Lys
GCC AAG TAT ATC TGT GAA AAT CAA GAT TCG ATC TCC AGT AAA

Leu Lys Glu Cys Cys Glu Lys Pro Leu Phe Glu Lys Ser His
CTG AAG GAA TGC TGT GAA AAA CCT CTG TTG GAA AAA TCC CAC

Cys Ile Ala Glu Val Glu Asn Asp Glu Met Pro Ala Asp Phe
TGC ATT GCC GAA GTG GAA AAT GAT GAG ATG CCT GCT GAC TTG

Pro Ser Phe Ala Val Asp Phe Val Glu Ser Lys Asp Val Cys
CCT TCA TTA GCT GCT GAT TTT GTT GAA AGT AAG GAT GTT TGC

Lys Asn Tyr Ala Glu Ala Lys Asp Val Phe Leu Gly Met Phe
AAA AAC TAT GCT GAG GCA AAG GAT GTC TTC CTG GGC ATG TTT

Phe Tyr Glu Tyr Ala Arg Arg His Pro Asp Tyr Ser Val Val
TTG TAT GAA TAT GCA AGA AGG CAT CCT GAT TAC TCT GTC GTG

Leu Leu Leu Arg Leu Ala Lys Thr Tyr Glu Thr Thr Leu Glu
CTG CTG CTG AGA CTT GCC AAG ACA TAT GAA ACC ACT CTA GAG

Lys Cys Cys Ala Ala Ala Asp Pro His Glu Cys Tyr Ala Lys
AAG TGC TGT GCC GCT GCA GAT CCT CAT GAA TGC TAT GCC AAA

Val Phe Asp Glu Phe Lys Pro Pro Val Glu Glu Pro Gln Asn
GTG TTC GAT GAA TTT AAA CCT CCT GTG GAA GAG CCT CAG AAT

Phe Ile Lys Gln Asn Cys Glu Leu Phe Glu Gln Leu Gly Glu
TTA ATC AAA CAA AAT TGT GAG CTT TTT GAG CAG CTT GGA GAG
```

Figure 2 (continued)

```
Tyr Lys Phe Gln Asn Ala Leu Phe Val Arg Tyr Thr Lys Lys
TAC AAA TTC CAG AAT GCG CTA TTA GTT CGT TAC ACC AAG AAA

Val Pro Gln Leu Ser Thr Pro Thr Leu Val Glu Val Ser Arg
GTA CCC CAA GTG TCA ACT CCA ACT CTT GTA GAG GTC TCA AGA

Asn Leu Gly Lys Val Gly Ser Lys Cys Cys Lys His Pro Glu
AAC CTA GGA AAA GTG GGC AGC AAA TGT TGT AAA CAT CCT GAA

Ala Lys Arg Met Pro Cys Ala Glu Asp Tyr Leu Ser Val Val
GCA AAA AGA ATG CCC TGT GCA GAA GAC TAT CTA TCC GTG GTC

Leu Asn Gln Leu Cys Val Leu His Glu Lys Thr Pro Val Ser
CTG AAC CAG TTA TGT GTG TTG CAT GAG AAA ACG CCA GTA AGT

Asp Arg Val Thr Lys Cys Cys Thr Glu Ser Leu Val Asn Arg
GAC AGA GTC ACC AAA TGC TGC ACA GAA TCC TTG GTG AAC AGG

Arg Pro Gly Phe Ser Ala Leu Glu Val Asp Glu Thr Tyr Val
CGA CCA TGC TTT TCA GCT CTG GAA GTC GAT GAA ACA TAC GTT

Pro Lys Glu Phe Asn Ala Glu Thr Phe Thr Phe His Ala Asp
CCC AAA GAG TTT AAT GCT GAA ACA TTC ACC TTC CAT GCA GAT

Ile Cys Thr Leu Ser Glu Lys Glu Arg Gln Ile Lys Lys Glu
ATA TGC ACA CTT TCT GAG AAG GAG AGA CAA ATC AAG AAA CAA

Thr Ala Leu Val Glu Leu Val Lys His Lys Pro Lys Ala Thr
ACT GCA CTT GTT GAG CTC GTG AAA CAC AAG CCC AAG GCA ACA

Lys Glu Glu Leu Lys Ala Val Met Asp Asp Phe Ala Ala Phe
AAA GAG CAA CTG AAA GCT GTT ATG GAT GAT TTC GCA GCT TTT

Val Glu Lys Cys Cys Lys Ala Asp Asp Lys Glu Thr Cys Phe
GTA GAG AAG TGC TGC AAG GCT GAC GAT AAG GAG ACC TGC TTC

Ala Glu Glu Gly Lys Lys Leu Val Ala Ala Ser Glu Ala Val
GCC GAG GAG GGT AAA AAA CTT GTT GCT GCA AGT CAA GCT GCC

Leu Gly Leu STOP
TTA GGC TTA TAA CATCTACATTTAAAAGCATCTCAGCCTACCATGAGAATA

AGAGAAAGAAAATGAAGATCAAAAGCTTATTCATCTGTTTTCTTTTTCGTTGGTG

TTTTAATCATTTTGCCTCTTTTCTCTGTGCTTCAATTAATAAAAAATGGAAAGAA

TCTAA
```

FIG. 3.

*FIG. 5.*

DIRECT NUCLEOTIDE SEQUENCE DETERMINATION FROM mRNA

(a) HL8
(LIVER FROM WHICH pGX 401 WAS DERIVED)

(b) (TWO DIFFERENT HUMAN LIVERS)
HL33    HL39

pGX 401
5′ GAA CCT GAG AGA AAT GAA TGC TTC
3′ CTT GGA CTC TCT TTA CTT ACG AAG
97

6/6

020 6733

0206733

# EUROPEAN SEARCH REPORT

**European Patent Office**

Application number

## DOCUMENTS CONSIDERED TO BE RELEVANT

EP 86304656.1

| Category | Citation of document with indication, where appropriate, of relevant passages | Relevant to claim | CLASSIFICATION OF THE APPLICATION (Int. Cl.4) |
|---|---|---|---|
| X | <u>EP - A2 - 0 079 739</u> (THE UPJOHN COMPANY) <br><br> * Abstract; claims 5-17 * <br><br> -- | 1-11, 13-17 | C 12 N 15/00 <br> C 07 K 13/00 <br> C 07 H 21/04 <br> C 12 N 1/20 |
| X | <u>EP - A2 - 0 073 646</u> (GENENTECH, INC.) <br><br> * Claims 1,3,6,7,9,10,12,14,15; fig. 3 * <br><br> -- | 1-11, 13-17 | C 12 P 21/02 <br> C 12 R 1:19 <br> C 12 R 1:185 |
| X | <u>EP - A2 -0 091 527</u> (PRESIDENT AND FELLOWS OF HARVARD COLLEGE) <br><br> * Claims 1-8,14-16; fig. 4 * <br><br> ---- | 1-4,6, 9-17 | |

|  |
|---|
| **TECHNICAL FIELDS SEARCHED (Int. Cl.4)** |
| C 12 N <br> C 07 K <br> C 07 H <br> C 12 P |

The present search report has been drawn up for all claims

| Place of search | Date of completion of the search | Examiner |
|---|---|---|
| VIENNA | 25-08-1986 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82